# EUROPÄISCHE PATENTANMELDUNG

(11) **EP 3 875 597 A1**
(43) Veröffentlichungstag der Anmeldung: **08.09.2021**
(21) Anmeldenummer: 20161537.4
(22) Anmeldetag: 06.03.2020
(51) Int. Cl.: C12P 33/06

(54) **VERFAHREN ZUR HYDROXYLIERUNG VON STEROIDEN**

(71) Anmelder: Annikki GmbH, 8074 Raaba-Grambach (AT); Pharmazell GmbH, 83064 Raubling (DE)
(72) Erfinder: STAUNIG, Nicole, 8076 Vasoldsberg (AT); DONSBACH, Kai Oliver, 83278 Traunstein (DE)
(74) Vertreter: Pföstl, Andreas

(57) **Zusammenfassung**

Die vorliegende Patentanmeldung betrifft ein Verfahren zur Herstellung eines Steroids umfassend den Schritt des Umsetzens eines 7-Desoxysteroids mit einem Cytochrom P450-Enzym oder einer funktionellen Variante davon in Anwesenheit mindestens eines Ferredoxins umfassend NADH/NAD⁺ und/oder NADPH/NADP⁺ als Kofaktor und mindestens einer Ferredoxin-Reduktase, wobei der Kofaktor in Anwesenheit mindestens einer Oxidoreduktase und mindestens einem Substrat der mindestens einen Oxidoreduktase regeneriert wird.

## Beschreibung

### TECHNISCHES GEBIET

Die vorliegende Erfindung betrifft Mittel und Verfahren zur Hydroxylierung von Steroiden.

### HINTERGRUND DER ERFINDUNG

3,7,12-Trihydroxylierte Gallensäuren, wie z.B. Cholsäure (3α,7α,12α-Trihydroxy-5β-cholansäure) oder Ursocholsäure (3α,7β,12α-Trihydroxy-5β-cholansäure) sind industriell bedeutsame Chemikalien, unter anderem als Ausgangsstoffe zur Herstellung von Ursodesoxycholsäure (UDCA). Ursodesoxycholsäure wird als Medikament u.a. eingesetzt zur Auflösung kleinerer Röntgen-negativer Gallensteine sowie zur Behandlung der Lebererkrankungen primär ziliare Zirrhose und primär sklerosierende Cholangitis.

Die industriell wichtigste Quelle für 3,7,12-trihydroxylierte Gallensäuren ist Gallenflüssigkeit aus Gallenblasen, die als Schlachtabfälle in der Fleischproduktion anfallen. Neben anderen Tierarten wird häufig die Galle von Rindern verwendet. Es existiert keine industriell relevante Totalsynthese für 3,7,12-trihydroxylierte Gallensäuren. Da die Produktion von Gallensäuren an ein anderes Produkt (Fleisch) gekoppelt ist, kann nur sehr begrenzt auf eine gesteigerte Nachfrage reagiert werden. Aus diesem Grund ist eine möglichst effiziente Nutzung des Rohstoffs Galle von großem Interesse.

Da Galle eine wässrige Mischung aus Gallensäuren, Lipiden, Cholesterin und anderen Substanzen ist, kommt der Trennung der Bestandteile bei der Gewinnung von Gallensäuren eine besondere Bedeutung zu. Auch die Gallensäuren stellen wiederum ein Gemisch dar, dessen Bestandteile sich durch Anzahl und Position der Hydroxylgruppen unterscheiden. In Rindergalle ist neben Cholsäure auch ein signifikanter Anteil von Desoxycholsäure enthalten, welche sich von Cholsäure durch das Fehlen der OH-Gruppe an Position 7 unterscheidet (3α,12α-Dihydroxy-5β-cholansäure). Desoxycholsäure hat einen wesentlich geringeren kommerziellen Wert als 3,7,12-trihydroxylierte Gallensäuren. Es besteht daher ein industrielles Interesse an der Überführung von Desoxycholsäure in eine 3,7,12-trihydroxylierte Gallensäure durch gezielte Einführung einer Hydroxylgruppe an Position 7.

Bei Hydroxylierungen wird formal in einer Oxidationsreaktion ein Sauerstoff-Atom in eine (nicht aktivierte) C-H-Bindung eingefügt. In der organischen Chemie sind dies sehr schwierig durchzuführende Reaktionen. Häufig werden OH-Gruppen durch Umwege eingefügt, z.B. durch die Addition von Wasser an einer C=C-Doppelbindung. Die selektive Hydroxylierung an einer bestimmten Position eines komplexen Moleküls (wie z.B. einer Gallensäure) ist problematisch, da mehrere chemisch (nahezu) gleichwertige C-H-Bindungen vorliegen.

Es ist daher eine Aufgabe der vorliegenden Erfindung Mittel und Verfahren bereitzustellen, Steroide, wie Gallensäuren und Derivate davon, welche an Position 7 ein Wasserstoff und keine Hydroxylgruppe aufweisen, spezifisch an dieser Stelle zu hydroxylieren.

### ZUSAMMENFASSUNG DER ERFINDUNG

Die erfindungsgemäße Aufgabe wird durch ein Verfahren zur Herstellung eines Steroids mit der allgemeinen Formel (I) 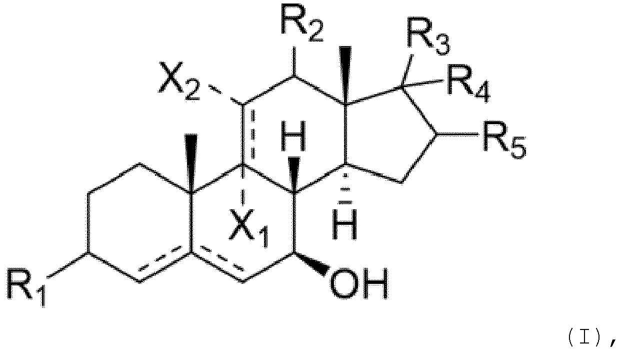 wobei
- X₁ und X₂: unabhängig voneinander H, Cl, F, Br, I, CF₃, ein C₁ bis C₆ Alkylrest, OH, ein C₁ bis C₆ Alkoxyrest, CN, NO₂, N(R₆)₂, eine Epoxygruppe, CHO oder ein CO₂R₆-Rest sind, wobei
R₆-C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph,-C(O)CH₂Ph ist,
- R₁ und R₂: unabhängig voneinander H, OH, OR₇ oder O sind, wobei
R₇ -C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph,-C(O)CH₂Ph ist,
- R₃: H, OH, OR₈, ein C₁ bis C₁₀ Alkylrest, ein C₁ bis C₁₀ Alkenylrest, -CHO, -C(O)(CH₃), -C(O)(CH₂OH),-CH(CH₃)C(O)CH₃, -CH (CH₃)((CH₂)₂CO₂R₉) oder-CH(CH₃)((CH₂)₂CONHR₉) ist, wobei
R₈-C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph, oder-C(O)CH₂Ph ist, und
R₉-CH₃, -CH₂COOH, -CH₂CH₃, -CH(CH₃)₂, -(CH₂)₂CH₃,-(CH₂)₂SO₃H, C (CH₃)₃, -(CH₂)₃CH₃, -CH(CH₃)CH₂CH₃,-CH₂CH₂(CH₃)₂, eine Arylgruppe oder eine Alkylaryl-gruppe ist,
- R₄: H, OH, oder -OR₁₀ ist, wobei
R₁₀ -C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph, oder-C(O)CH₂Ph ist und
- R₅: H, CF₃, ein C₁ bis C₆ Alkylrest, ein C₁ bis C₆ Alkenyl-rest, OH, O, oder ein C₁ bis C₆ Alkoxyrest ist, wobei
die gestrichelte Linie eine optionale Doppelbindung bedeutet, mit der Maßgabe, dass der B-Ring keine Doppelbindung aufweist, wenn der A-Ring eine C4-C5-Doppelbindung und der C-Ring keine Doppelbindung aufweist, wenn X₁ und X₂ eine Epoxygruppe ausbilden,
umfassend den Schritt des Umsetzens eines 7-Desoxysteroids mit der allgemeinen Formel (II) 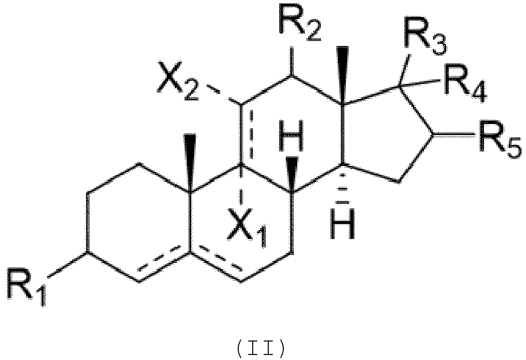 mit einer Cytochrom P450- Hydroxylase oder einer funktionellen Variante davon in Anwesenheit mindestens eines Redoxpartnersystems und eines Systems zur Regenerierung des Redoxpartnersystems, gelöst.

Es hat sich überraschenderweise gezeigt, dass Cytochrom P450 und funktionelle Fragmente davon in der Lage sind, Steroide, wie Cholsäure bzw. Derivate davon mit der Formel (I) an Position 7 zu hydroxylieren. Durch die Kopplung dieser Reaktion mit mindestens eines Redoxpartnersystems und eines Systems zur Regenerierung des Redoxpartnersystems kann das Gleichgewicht der Reaktion auf die Seite des Endprodukts verschoben und somit dessen Ausbeute signifikant gesteigert werden. Die Regeneration des Redoxpartnersystems kann dabei in Anwesenheit eines zweiten Systems (eines Regenerationssystems) erfolgen, welches vorzugsweise mindestens eine Oxidoreduktase und mindestens ein Substrat der mindestens einen Oxidoreduktase umfasst.

### BESCHREIBUNG DER AUSFÜHRUNGSFORMEN

Cytochrom P450 und funktionelle Varianten davon, die die Oxidation von Reduktionsäquivalenten NAD(P)H erfordern, sind überraschender Weise in der Lage, 7-Desoxysteroide, wie z.B. 7-Desoxycholsäure, und Derivate davon selektiv an Position 7 zu hydroxylieren.

Cytochrome P450 katalysieren Monooxygenase-Reaktionen einer Vielzahl von endogenen sowie von exogenen Substraten. Sie sind u.a. am Stoffwechsel von Steroiden, Eicosanoiden, Fettsäuren und Gallensäuren sowie von exogenen Substraten, wie Arzneistoffen, Insektiziden und chemischen Karzinogenen beteiligt.

Cytochrome P450 gemäß vorliegender Erfindung können beispielweise aus Bakterien, wie z.B. Actinobakterien, insbesondere z.B. der Gattung Streptomyces verwendet werden. Dabei können die Sequenzen mit Hilfe bekannter Techniken zum Beispiel aus genomischer DNA oder einer cDNA-Bibliothek isoliert werden.

Die Cytochrome P450 gemäß vorliegender Erfindung bzw. deren funktionelle Varianten können wahlweise in ihrem Ursprungsorganismus vorhanden oder aus diesem isoliert sein, oder sie sind rekombinant exprimiert oder synthetisch hergestellt. Vorzugsweise werden erfindungsgemäß rekombinant exprimierte Polypeptide verwendet.

Zur rekombinanten Expression von Enzymen gemäß der vorliegenden Erfindung können verschiedene etablierte Mikroorganismen verwendet werden, wie z.B. Escherichia coli (E. coli), *Bacillus subtilis, Saccharomyces cerevisiae* oder *Pichia pastoris.* Entsprechende Protokolle sind dazu sind in der einschlägigen Fachliteratur ausführlich beschrieben oder einer fachkundigen Person bekannt.

Enzyme/Polypeptide werden gemäß vorliegender Erfindung bevorzugt als in *E. coli* rekombinant überexprimierte Proteine verwendet, wobei bevorzugt die entsprechenden Zell-Lysate entweder ohne weitere Bearbeitung/Aufreinigung oder nach relativ einfachen Bearbeitungsschritten (z.B. Zentrifugation, Fällung, Konzentration oder Gefriertrocknung) eingesetzt werden. Alternativ können auch *E. coli* Zellen nach rekombinanter Überexpression der verwendeten Enzyme ohne Zellaufschluss direkt oder zum Beispiel nach einem Zyklus Einfrieren/Auftauen in der Reaktion eingesetzt werden. Geeignete Expressionsplasmide sind einer fachkundigen Person bekannt und können vielfach kommerziell erworben werden.

"Funktionelle Varianten" von Cytochrom P450 können Fragmente oder Mutationsvarianten von Cytochrom P450 sein, wobei Fragmente von Cytochrom P450 auch als "funktionelle Fragmente" bezeichnet werden können. "Funktionelle Varianten" von Cytochrom P450 sind in der Lage dieselbe Reaktion zu katalysieren, wie das Protein, von dem diese abgeleitet wurden. Ob eine Variante funktionell ist, d.h. ob diese dieselbe Reaktion katalysiert wie das Protein, von dem diese abgeleitet wird, kann dadurch bestimmt werden, in dem festgestellt wird, dass die Variante dieselbe Reaktion katalysiert. Hierfür gibt es etablierte Methoden im Stand der Technik bzw. jene, die hierin beschrieben sind. Die Umsetzungsraten von Substraten durch die erfindungsgemäßen funktionellen Varianten können von den Umsetzungsraten des Cytochroms P450, von dem diese abgeleitet wurden, abweichen.

"Derivate von 7-Desoxysteroiden" umfassen von 7-Desoxysteroiden abgeleitete Verbindungen mit unterschiedlichsten Modifikationen wie oben definiert.

Gemäß einer bevorzugten Ausführungsform der vorliegenden Erfindung sind X₁, X₂, R₄ und R₅ H und
- R₁ und R₂: unabhängig voneinander H, OH, OR₈ oder O, wobei R₈-C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph, -C(O)CH₂Ph ist,
- R₃: ein C₁ bis C₁₀ Alkylrest, ein C₁ bis C₁₀ Alkylenrest,-CH(CH₃)((CH₂)₂CO₂R₉) oder -CH (CH₃)((CH₂)₂CONHR₉), wobei
R₉-CH₃, -CH₂COOH, -CH₂CH₃, -CH(CH₃)₂, -(CH₂)₂CH₃,-(CH₂)₂SO₃H, C (CH₃)₃, - (CH₂)₃CH₃, -CH (CH₃)CH₂CH₃,-CH₂CH₂(CH₃)₂, eine Arylgruppe oder eine Alkylarylgruppe ist.

Gemäß einer weiteren bevorzugten Ausführungsform der vorliegenden Erfindung ist die Arylgruppe ausgewählt aus der Gruppe bestehend aus einem Phenylrest, einem mit F, Cl, Br, NO₂ oder CH₃ substituierten Phenylrest und einem Heteroaryl.

Gemäß einer noch weiteren bevorzugten Ausführungsform der vorliegenden Erfindung ist die Alkylarylgruppe ausgewählt aus der Gruppe bestehend aus einer Benzylgruppe, einer halogenierten Benzylgruppe, wobei das Halogen F, Cl oder Br ist, und einer mit NO₂ substituierten Benzylgruppe.

Gemäß einer bevorzugten Ausführungsform der vorliegenden Erfindung ist R₁CH(CH₃)((CH₂)₂CO₂R₅), R₂H, R₃O oder OH, R₄OH und R₅H.

Gemäß einer weiteren bevorzugten Ausführungsform der vorliegenden Erfindung ist das 7-Desoxysteroid mit der allgemeinen Formel (II) ausgewählt aus der Gruppe bestehend aus 3α,12α-Dihydroxy-5β-cholan-24-Säure, 3α,12β-Dihydroxy-5β-cholan-24-Säure, 3β,12α-Dihydroxy-5β-cholan-24-Säure, 3β,12β-Dihydroxy-5β-cholan-24-Säure, 3β-Hydroxy-12-Keto-5β-cholan-24-Säure, 3-Keto,12β-hydroxy-5β-cholan-24-Säure, 3-Keto,12α-hydroxy-5β-cholan-24-Säure, 3α-Hydroxy-5β-cholan-24-Säure, 3-Keto-5β-cholan-24-Säure, 3β-Hydroxy-5β-cholan-24-Säure und Ester der jeweiligen Säure.

Das Cytochrom P450-Enzym, das erfindungsgemäß zur Hydroxylierung von 7-Desoxysteroiden und Derivaten davon mit der allgemeinen Formel (II) zu einem Steroid oder einem Derivat davon mit der allgemeinen Formel (I) eingesetzt wird, umfasst vorzugsweise eine Aminosäuresequenz auf, die mindestens 80%, vorzugsweise mindestens 85%, noch mehr bevorzugt mindestens 90%, noch mehr bevorzugt mindestens 95%, noch mehr bevorzugt mindestens 96%, noch mehr bevorzugt mindestens 97%, noch mehr bevorzugt mindestens 98%, noch mehr bevorzugt mindestens 99%, insbesondere 100%, ident ist mit der Aminosäuresequenz SEQ ID Nr. 1 oder 2 ist.

SEQ ID Nr. 1:

MLTTAETTSIAYPFNTAEGLALSERYEEARNRTGLLRVRMPYGEPAWLVTRYADARLVLGDR
RFSRAEALHHDEPRQSEGRRDSGILTMDPPDHTRLRTLVAKAFTVHQVEKLRPWVRQLTHDL
LDDLEAAGPPADLVDRYALPIPVGVICAMLGVPQEDRPKFRVWSDAALSTSSLSAEQFARNT
DELRAYMAGLIEDHRRTPRDDIMTSLIEARDAGDRLSELELVDLCVGILVAGHETTATQIPN
FVLTLLEHPDQLRRLREDPALIQGAVEELLRFVPLGVGAAQARYATEDIEVGGTLVRSGEPV
LVAVGSANRDALRFDEPGVLNVARPTTQHLGFGHGVHHCLGAPLARLELQEALGALITRFPG
LRLAGDIEWKDRMLVRGPRVMPIGW

SEQ ID Nr. 2:

MPYGEPAWLVTRYADARLVLGDRRFSRAEALHHDEPRQSEGRRDSGILTMDPPDHTRLRTLV
AKAFTVHQVEKLRPWVRQLTHDLLDDLEAAGPPADLVDRYALPIPVGVICAMLGVPQEDRPK
FRVWSDAALSTSSLSAEQFARNTDELRAYMAGLIEDHRRTPRDDIMTSLIEARDAGDRLSEL
ELVDLCVGILVAGHETTATQIPNFVLTLLEHPDQLRRLREDPALIQGAVEELLRFVPLGVGA
AQARYATEDIEVGGTLVRSGEPVLVAVGSANRDALRFDEPGVLNVARPTTQHLGFGHGVHHC
LGAPLARLELQEALGALITRFPGLRLAGDIEWKDRMLVRGPRVMPIGW

Aminosäuresequenzen SEQ ID Nr. 1 und 2 werden vorzugsweise durch Nukleinsäuresequenzen SEQ ID Nr. 3 und 4 kodiert, wobei Nukleinsäuresequenzen SEQ ID Nr. 5 und 6 für die Expression in *E.coli* optimiert sind.

SEQ ID Nr. 3:

ATGTTGACCACAGCCGAGACGACATCCATCGCCTATCCCTTCAACACCGCCGAAGGCTGGC
GCTCAGCGAGCGTTACGAAGAGGCCAGGAACCGCACCGGACTGCTCCGGGTGCGGATGCCCT
ACGGTGAGCCCGCCTGGCTGGTCACGCGGTACGCCGACGCCCGGCTGGTGCTCGGCGACCGG
CGCTTCAGCCGTGCGGAGGCGCTCCACCACGACGAGCCGCGGCAGTCCGAAGGCCGGCGCGA
CAGCGGCATCCTGACCATGGACCCGCCCGACCACACCCGGCTGCGCACCCTCGTCGCCAAGG
CGTTCACCGTCCACCAGGTGGAGAAACTCCGCCCCTGGGTACGCCAGTTGACCCATGACCTG
CTCGACGACCTCGAGGCCGCCGGGCCGCCCGCCGATCTGGTGGACCGCTACGCCCTGCCCAT
TCCGGTCGGCGTCATCTGCGCCATGCTCGGCGTCCCGCAGGAGGACCGGCCCAAGTTCCGGG
TCTGGAGCGACGCCGCGCTGTCCACCAGCTCGCTGAGCGCCGAGCAGTTCGCCCGTAACACC
GACGAGCTGCGCGCCTACATGGCCGGGCTGATCGAGGACCACCGCAGGACCCGCGGACGA
CATCATGACCTCGCTGATCGAGGCGCGGACGCGGGCGACCGGCTGTCCGAGCTGGAACTCG
TCGATCTGTGCGTGGGCATCCTGGTGGCCGGGCACGAGACCACCGCCACCCAGATCCCCAAC
TTCGTGCTGACGCTGCTGGAGCACCCGGACCAGCTGCGCCGGCTGCGCGAGGACCCCGCCCT
GATCCAGGGCGCCGTCGAGGAGCTGCTGCGCTTCGTCCCGCTGGGCGTGGGCGCCGCCCAGG

CCCGTTACGCCACCGAGGACATCGAGGTGGGCGGCACGCTGGTGCGCAGCGGGAGCCGGTG
CTGGTCGCCGTCGGCTCGGCCAACCGCGACGCGCTGCGCTTCGACGAACCGGGCGTGCTCAA
CGTCGCCCGCCCCACCACCCAGCACCTCGGCTTCGGCCACGGTGTGCACCACTGCCTGGGCG
CGCCCCTGGCCCGTCTGGAGCTCCAGGAGGCGCTCGGCGCGCTGATCACGCGCTTCCCGGGC
CTGCGGCTGGCCGGGGACATCGAGTGGAAGGACCGCATGCTGGTCCGCGGCCCCGTGTCAT
GCCCATCGGGTGGTGA

SEQ ID Nr. 4:

ATGCCCTACGGTGAGCCCGCCTGGCTGGTCACGCGGTACGCCGACGCCCGGCTGGTGCTCGG
CGACCGGCGCTTCAGCCGTGCGGAGGCGCTCCACCACGACGAGCCGCGGCAGTCCGAAGGCC
GGCGCGACAGCGGCATCCTGACCATGGACCCGCCCGACCACACCCGGCTGCGCACCCTCGTC
GCCAAGGCGTTCACCGTCCACCAGGTGGAGAAACTCCGCCCCTGGGTACGCCAGTTGACCCA
TGACCTGCTCGACGACCTCGAGGCCGCCGGGCCGCCCGCCGATCTGGTGGACCGCTACGCCC
TGCCCATTCCGGTCGGCGTCATCTGCGCCATGCTCGGCGTCCCGCAGGAGGACCGGCCCAAG
TTCCGGGTCTGGAGCGACGCCGCGCTGTCCACCAGCTCGCTGAGCGCCGAGCAGTTCGCCCG
TAACACCGACGAGCTGCGCGCCTACATGGCCGGGCTGATCGAGGACCACCGCAGGACCCCGC
GGGACGACATCATGACCTCGCTGATCGAGGCGCGGACGCGGGCGACCGGCTGTCCGAGCTG
GAACTCGTCGATCTGTGCGTGGGCATCCTGGTGGCCGGGCACGAGACCACCGCCACCCAGAT
CCCCAACTTCGTGCTGACGCTGCTGGAGCACCCGGACCAGCTGCGCCGGCTGCGCGAGGACC
CCGCCCTGATCCAGGGCGCCGTCGAGGAGCTGCTGCGCTTCGTCCCGCTGGGCGTGGGCGCC
GCCCAGGCCCGTTACGCCACCGAGGACATCGAGGTGGGCGGCACGCTGGTGCGCAGCGGGGA
GCCGGTGCTGGTCGCCGTCGGCTCGGCCAACCGCGACGCGCTGCGCTTCGACGAACCGGGCG
TGCTCAACGTCGCCCGCCCCACCACCCAGCACCTCGGCTTCGGCCACGGTGTGCACCACTGC
CTGGGCGCGCCCCTGGCCCGTCTGGAGCTCCAGGAGGCGCTCGGCGCGCTGATCACGCGCTT
CCCGGGCCTGCGGCTGGCCGGGGACATCGAGTGGAAGGACCGCATGCTGGTCCGCGGCCCC
GTGTCATGCCCATCGGGTGGTGA

SEQ ID Nr. 5:

ATGCTGACCACCGCAGAAACCACCAGTATTGCATATCCGTTTAATACCGCAGAAGGTCTGGC
ACTGAGCGAACGTTATGAAGAAGCACGTAATCGTACCGGTCTGCTGCGTGTTCGTATGCCGT
ATGGTGAACCGGCATGGCTGGTTACCCGTTATGCAGATGCCCGTCTGGTTCTGGGTGATCGT
CGTTTTAGCCGTGCCGAAGCACTGCATCACGATGAACCGCGTCAGAGCGAAGGTCGTCGTGA
TAGCGGTATTCTGACCATGGATCCGCCTGATCATACCCGTCTGCGTACCCTGGTTGCAAAAG
CATTTACCGTTCATCAGGTTGAAAAACTGCGTCCGTGGGTTCGCCAGCTGACCCATGATCTG
CTGGATGATCTGGAAGCAGCAGGTCCGCCTGCAGATCTGGTTGATCGTTATGCACTGCCGAT
TCCGGTTGGTGTTATTTGTGCAATGCTGGGTGTTCCGCAAGAAGATCGTCCTAAATTTCGTG

TTTGGAGTGATGCAGCACTGAGCACCAGCAGCCTGAGCGCAGAACAGTTTGCACGTAATACC
GATGAACTGCGTGCATATATGGCAGGTCTGATTGAAGATCATCGTCGTACACCGCGTGATGA
TATTATGACCAGCCTGATCGAAGCACGTGATGCCGGTGATCGCCTGAGTGAACTGGAACTGG
TGGATCTGTGTGTTGGTATTCTGGTTGCAGGTCATGAAACCACCGCAACCCAGATTCCGAAT
TTTGTTCTGACCCTGCTGGAACATCCGGATCAGCTGCGTCGTCTGCGTGAAGATCCGGCACT
GATTCAGGGTGCAGTTGAAGAACTGCTGCGTTTTGTTCCGCTGGGTGTGGGTGCAGCACAGG
CACGTTATGCAACCGAAGATATTGAAGTTGGTGGCACCCTGGTTCGTAGTGGCGAACCGGTG
CTGGTTGCCGTTGGTAGCGCAAACCGTGATGCACTGCGCTTTGATGAACCGGGTGTTCTGAA
TGTTGCACGTCCGACCACACAGCATCTGGGTTTTGGTCATGGTGTTCATCATTGTCTGGGTG
CACCGCTGGCACGTCTGGAACTGCAAGAAGCACTGGGAGCACTGATTACCCGTTTTCCGGGT
CTGCGTCTGGCAGGCGATATTGAATGGAAAGATCGTATGCTGGTTCGTGGTCCGCGTGTTAT
GCCGATTGGTTGGTAA

SEQ ID Nr. 6:

ATGGTGAACCGGCATGGCTGGTTACCCGTTATGCAGATGCCCGTCTGGTTCTGGGTGATCGT
CGTTTTAGCCGTGCCGAAGCACTGCATCACGATGAACCGCGTCAGAGCGAAGGTCGTCGTGA
TAGCGGTATTCTGACCATGGATCCGCCTGATCATACCCGTCTGCGTACCCTGGTTGCAAAAG
CATTTACCGTTCATCAGGTTGAAAAACTGCGTCCGTGGGTTCGCCAGCTGACCCATGATCTG
CTGGATGATCTGGAAGCAGCAGGTCCGCCTGCAGATCTGGTTGATCGTTATGCACTGCCGAT
TCCGGTTGGTGTTATTTGTGCAATGCTGGGTGTTCCGCAAGAAGATCGTCCTAAATTTCGTG
TTTGGAGTGATGCAGCACTGAGCACCAGCAGCCTGAGCGCAGAACAGTTTGCACGTAATACC
GATGAACTGCGTGCATATATGGCAGGTCTGATTGAAGATCATCGTCGTACACCGCGTGATGA
TATTATGACCAGCCTGATCGAAGCACGTGATGCCGGTGATCGCCTGAGTGAACTGGAACTGG
TGGATCTGTGTGTTGGTATTCTGGTTGCAGGTCATGAAACCACCGCAACCCAGATTCCGAAT
TTTGTTCTGACCCTGCTGGAACATCCGGATCAGCTGCGTCGTCTGCGTGAAGATCCGGCACT
GATTCAGGGTGCAGTTGAAGAACTGCTGCGTTTTGTTCCGCTGGGTGTGGGTGCAGCACAGG
CACGTTATGCAACCGAAGATATTGAAGTTGGTGGCACCCTGGTTCGTAGTGGCGAACCGGTG
CTGGTTGCCGTTGGTAGCGCAAACCGTGATGCACTGCGCTTTGATGAACCGGGTGTTCTGAA
TGTTGCACGTCCGACCACACAGCATCTGGGTTTTGGTCATGGTGTTCATCATTGTCTGGGTG
CACCGCTGGCACGTCTGGAACTGCAAGAAGCACTGGGAGCACTGATTACCCGTTTTCCGGGT
CTGCGTCTGGCAGGCGATATTGAATGGAAAGATCGTATGCTGGTTCGTGGTCCGCGTGTTAT
GCCGATTGGTTGGTAA

"Ident", wie hier verwendet, bedeutet, dass zwei oder mehr Aminosäuresequenzen, wenn diese übereinander gelagert werden, eine bestimmte "Identität" (übereinstimmende Aminosäurereste an identen Positionen) zueinander aufweisen können. "Identität" wird in dieser Erfindung definiert als die Prozentzahl der Aminosäuren der in Frage kommenden Aminosäuresequenzen, die identisch mit den Aminosäuren der Ausgangssequenz sind, und zwar nach Abgleich der beiden Sequenzen und der Einführung von Lücken, falls notwendig, um die maximale prozentuale Sequenzidentität zu erzielen wie sie durch das Programm "Protein BLAST" (blastp; Altschul et al., J. Mol. Biol. (1997) 215:403-410; http://blast.ncbi.nlm.nih.gov/Blast.cgi; hier im Allgemeinen als "BLAST" bezeichnet) erzeugt werden, wobei alle variablen Parameter auf Default-Werte gesetzt sind. Ein prozentualer (%) Wert für die Aminosäuresequenzidentität wird durch die Zahl der übereinstimmenden identischen Nukleotide dividiert durch die Sequenzlänge, für welche die prozentuale Identität festgehalten wird, bestimmt.

Mit Hilfe des erfindungsgemäßen Verfahrens können 7-Desoxysteroide bzw. Derivate davon mit der allgemeinen Formel (II) mit Cytochrom P450 oder einer funktionellen Variante davon zu Steroiden bzw. Derivaten davon mit der allgemeinen Formel (I) umgesetzt werden. Diese Umsetzung erfolgt in Anwesenheit von Redoxpartnern oder eines Redoxpartnersystems, welches in der Lage ist Elektronen für die Hydroxylierungsreaktion zur Verfügung zu stellen.

Gemäß einer bevorzugten Ausführungsform der vorliegenden Erfindung umfasst das mindestens eine Redoxpartnersystem
(i) Ferredoxin, Ferredoxin-Reduktase und NAD(P)H;
(ii) Cytochrom P450 Reduktase und NAD(P)H; oder
(iii) NAD(P)H.

Das erfindungsgemäß verwendete Redoxpartnersystem kann Ferredoxin, Ferredoxin-Reduktase und NAD(P)H; Cytochrom P450 Reduktase und NAD(P)H; oder NAD(P)H, alleine, umfassen, wobei ein Redoxpartnersystem umfassend Ferredoxin, Ferredoxin-Reduktase und NAD(P)H besonders bevorzugt ist.

Um die Redoxreaktion von Cytochrom P450 bzw. deren funktionellen Varianten davon durchzuführen ist es somit von Vorteil im erfindungsgemäßen Verfahren zumindest die Redoxcofaktoren NAD+/NADH und/oder NADP+/NADPH einzusetzen. Dabei bezeichnet NAD+ die oxidierte Form und NADH die reduzierte Form von Nicotinamidadenindinucleotid, während NADP+ die oxidierte Form und NADPH die reduzierte Form von Nicotinamidadenindinucleotidphosphat bezeichnet.

Die Konzentration der Redoxcofaktoren NAD(P)+ und/oder NAD(P)H in einer Reaktionsmischung beträgt vorzugsweise zwischen 0,001 mM und 10 mM, noch mehr bevorzugt zwischen 0,05 mM und 1 mM.

Besonders bevorzugt werden Ferredoxine als Redoxpartner eingesetzt, welche in Anwesenheit von NAD(P)+ und mindestens einer Ferredoxin-Reduktase regeneriert werden können. Gemäß einer bevorzugten Ausführungsform der vorliegenden Erfindung ist das mindestens eine Ferredoxin ausgewählt aus der Gruppe bestehend aus Adrenodoxine, Putidaredoxine und Flavodoxine, wobei gegebenenfalls auch Kombinationen davon eingesetzt werden können.

Ein mögliches Paar von Redoxpartnern umfasst vorzugsweise Putidaredoxin und Putidaredoxin-Reduktase aus *Pseudomonas putida.* Eine fachkundige Person ist darüber hinaus in der Lage, weitere Ferredoxin-Proteine und Ferredoxin-Reduktasen zu identifizieren, welche potentielle Redoxpartner für das erfindungsgemäße Cytochrom P450 sind. Die Eignung als Redoxpartner lässt sich in einem funktionellen Assay überprüfen, wie beispielsweise in den Beispielen 3 bis 5 beschrieben wird. Das in diesen Beispielen verwendete Putidaredoxin und/oder die darin verwendete Putidaredoxin-Reduktase kann durch mögliche alternative Proteine bzw. Enzyme ersetzt werden. Wird eine ausreichende Bildung des gewünschten Produkts (z.B. Ursocholsäure) beobachtet, so können die getesteten Redoxpartner als funktionelle Alternativen zu Putidaredoxin und/oder Putidaredoxin-Reduktase betrachtet werden.

Gemäß einer besonders bevorzugten Ausführungsform der vorliegenden Erfindung umfasst das im erfindungsgemäßen Verfahren verwendete Ferredoxin eine Aminosäuresequenz auf, die mindestens 80%, vorzugsweise mindestens 85%, noch mehr bevorzugt mindestens 90%, noch mehr bevorzugt mindestens 95%, noch mehr bevorzugt mindestens 96%, noch mehr bevorzugt mindestens 97%, noch mehr bevorzugt mindestens 98%, noch mehr bevorzugt mindestens 99%, insbesondere 100%, ident ist mit der Aminosäuresequenz SEQ ID Nr. 7, wobei X ein Methionin-Rest oder keine Aminosäure ist.

SEQ ID Nr. 7:

XSKVVYVSHDGTRRELDVADGVSLMQAAVSNGIYDIVGDCGGSASCATCHVYVNEAFTDK
VPAANEREIGMLECVTAELKPNSRLCCQIIMTPELDGIVVDVPDRQW

Gemäß einer bevorzugten Ausführungsform der vorliegenden Erfindung ist die mindestens eine Ferredoxin-Reduktase ausgewählt aus der Gruppe der Flavodoxin-Reduktasen und Putidaredoxin-Reduktase.

Das im Zuge der erfindungsgemäßen Hydroxylierungsreaktion oxidierte Ferredoxin kann mit Hilfe einer Ferredoxin-Reduktase und NAD(P)H reduziert werden. Dadurch wird reduziertes Ferredoxin unter Verbrauch von NAD(P)H für eine weitere Hydroxylierungsreaktion des erfindungsgemäßen Substrats wieder bereitgestellt bzw. regeneriert. Die Ferredxon-Reduktase kann eine Flavodoxin-Reduktase und/oder eine Putidaredoxin-Reduktase sein.

Gemäß einer weiteren bevorzugten Ausführungsform der vorliegenden Erfindung umfasst das im erfindungsgemäßen Verfahren verwendete Ferredoxin-Reduktase eine Aminosäuresequenz auf, die mindestens 80%, vorzugsweise mindestens 85%, noch mehr bevorzugt mindestens 90%, noch mehr bevorzugt mindestens 95%, noch mehr bevorzugt mindestens 96%, noch mehr bevorzugt mindestens 97%, noch mehr bevorzugt mindestens 98%, noch mehr bevorzugt mindestens 99%, insbesondere 100%, ident ist mit der Aminosäuresequenz SEQ ID Nr. 8.

SEQ ID Nr. 8:

MNANDNVVIVGTGLAGVEVAFGLRASGWEGNIRLVGDATVIPHHLPPLSKAYLAGKATAE
SLYLRTPDAYAAQNIQLLGGTQVTAINRDRQQVILSDGRALDYDRLVLATGGRPRPLPVA
SGAVGKANNFRYLRTLEDAECIRRQLIADNRLVVIGGGYIGLEVAATAIKANMHVTLLDT
AARVLERVTAPPVSAFYEHLHREAGVDIRTGTQVCGFEMSTDQQKVTAVLCEDGTRLPAD
LVIAGIGLIPNCELASAAGLQVDNGIVINEHMQTSDPLIMAVGDCARFHSQLYDRWVRIE
SVPNALEQARKIAAILCGKVPRDEAAPWFWSDQYEIGLKMVGLSEGYDRIIVRGSLAQPD
FSVFYLQGDRVLAVDTVNRPVEFNQSKQIITDRLPVEPNLLGDESVPLKEIIAAAKAELS
SA

Aminosäuresequenzen SEQ ID Nr. 7 und 8 werden vorzugsweise durch Nukleinsäuresequenzen SEQ ID Nr. 9 bzw. 10 kodiert, wobei Nukleinsäuresequenzen SEQ ID Nr. 11 bzw. 12 für die Expression in *E.coli* optimiert sind.

SEQ ID Nr. 9:

(ATG)0 oder 1
TCTAAAGTAGTGTATGTGTCACATGATGGAACGCGTCGCGAACTGGATGTGGCGGATGGC
GTCAGCCTGATGCAGGCTGCAGTCTCCAATGGTATCTACGATATTGTCGGTGATTGTGGC
GGCAGCGCCAGCTGTGCCACCTGCCATGTCTATGTGAACGAAGCGTTCACGGACAAGGTG
CCCGCCGCCAACGAGCGGGAAATCGGCATGCTGGAGTGCGTCACGGCCGAACTGAAGCCG
AACAGCAGGCTCTGCTGCCAGATCATCATGACGCCCGAGCTGGATGGCATCGTGGTCGAT
GTTCCCGATAGGCAATGGTAA

SEQ ID Nr. 10:

ATGAACGCAAACGACAACGTGGTCATCGTCGGTACCGGACTGGCTGGCGTTGAGGTCGCC
TTCGGCCTGCGCGCCAGCGGCTGGGAAGGCAATATCCGGTTGGTGGGGGATGCGACGGTA
ATTCCCCATCACCTACCACCGCTATCCAAAGCTTACTTGGCCGGCAAAGCCACAGCGGAA
AGCCTGTACCTGAGAACCCCAGATGCCTATGCAGCGCAGAACATCCAACTACTCGGAGGC
ACACAGGTAACGGCTATCAACCGCGACCGACAGCAAGTAATCCTATCGGATGGCCGGGCA
CTGGATTACGACCGGCTGGTATTGGCTACCGGAGGGCGTCCAAGACCCCTACCGGTGGCC
AGTGGCGCAGTTGGAAAGGCGAACAACTTTCGATACCTGCGCACACTCGAGGACGCCGAG
TGCATTCGCCGGCAGCTGATTGCGGATAACCGTCTGGTGGTGATTGGTGGCGGCTACATT
GGCCTTGAAGTGGCTGCCACCGCCATCAAGGCGAACATGCACGTCACCCTGCTTGATACG
GCAGCCCGGGTTCTGGAGCGGGTTACCGCCCCGCCGGTATCGGCCTTTTACGAGCACCTA
CACCGCGAAGCCGGCGTTGACATACGAACCGGCACGCAGGTGTGCGGGTTCGAGATGTCG

ACCGACCAACAGAAGGTTACTGCCGTCCTCTGCGAGGACGGCACAAGGCTGCCAGCGGAT
CTGGTAATCGCCGGGATTGGCCTGATACCAAACTGCGAGTTGGCCAGTGCGGCCGGCCTG
CAGGTTGATAACGGCATCGTGATCAACGAACACATGCAGACCTCTGATCCCTTGATCATG
GCCGTCGGCGACTGTGCCCGATTTCACAGTCAGCTCTATGACCGCTGGGTGCGTATCGAA
TCGGTGCCCAATGCCTTGGAGCAGGCACGAAAGATCGCCGCCATCCTCTGTGGCAAGGTG
CCACGCGATGAGGCGGCGCCCTGGTTCTGGTCCGATCAGTATGAGATCGGATTGAAGATG
GTCGGACTGTCCGAAGGGTACGACCGGATCATTGTCCGCGGCTCTTTGGCGCAACCCGAC
TTCAGCGTTTTCTACCTGCAGGGAGACCGGGTATTGGCGGTCGATACAGTGAACCGTCCA
GTGGAGTTCAACCAGTCAAAACAAATAATCACGGATCGTTTGCCGGTTGAACCAAACCTA
CTCGGTGACGAAAGCGTGCCGTTAAAGGAAATCATCGCCGCCGCCAAAGCTGAACTGAGT
AGTGCCTGA

SEQ ID Nr. 11:

(ATG)0 oder 1
ATGAGCAAAGTGGTCTATGTGTCGCATGATGGAACACGCCGTGAGTTAGACGTCGCTGAT
GGTGTATCCCTGATGCAAGCAGCGGTTAGCAATGGCATTTACGACATCGTTGGCGATTGT
GGTGGTAGTGCGTCATGTGCAACGTGTCACGTGTATGTTAACGAAGCGTTTACCGATAAG
GTGCCTGCTGCCAATGAACGCGAGATTGGCATGCTGGAATGCGTAACTGCCGAACTCAAA
CCGAACTCTCGCCTGTGCTGCCAGATCATCATGACCCCGGAATTGGACGGGATTGTCGTT
GATGTGCCAGATCGTCAGTGGTAA

SEQ ID Nr. 12:

ATGAACGCCAATGATAATGTTGTTATTGTTGGCACCGGTCTGGCAGGCGTTGAAGTTGCA
TTTGGTCTGCGTGCAAGCGGTTGGGAAGGTAATATTCGTCTGGTTGGTGATGCAACCGTT
ATTCCGCATCATCTGCCTCCGCTGAGCAAAGCATATCTGGCAGGTAAAGCAACCGCAGAA
AGCCTGTATCTGCGTACACCGGATGCCTATGCAGCACAGAATATTCAGCTGCTGGGTGGT
ACACAGGTTACCGCAATTAATCGTGATCGTCAGCAGGTTATTCTGAGTGATGGTCGTGCA
CTGGATTATGATCGTCTGGTGCTGGCAACCGGTGGTCGTCCGCGTCCGCTGCCGGTTGCA
AGTGGTGCAGTTGGTAAAGCCAATAACTTTCGTTATCTGCGCACCCTGGAAGATGCAGAA
TGTATTCGTCGTCAGCTGATTGCAGATAATCGCCTGGTTGTGATTGGTGGTGGTTATATT
GGTCTGGAAGTTGCAGCAACCGCCATTAAAGCAAATATGCATGTTACCCTGCTGGATACC
GCAGCACGTGTTCTGGAACGTGTTACCGCACCGCCTGTTAGCGCCTTTTATGAACATCTG
CATCGTGAAGCCGGTGTTGATATTCGTACCGGCACCCAGGTTTGTGGTTTTGAAATGAGC
ACCGATCAGCAGAAAGTTACCGCAGTTCTGTGTGAAGATGGCACCCGTCTGCCTGCAGAT
CTGGTTATTGCAGGTATTGGCCTGATTCCGAATTGTGAACTGGCAAGCGCAGCAGGTCTG
CAGGTTGATAATGGTATTGTTATTAACGAACACATGCAGACCAGCGATCCGCTGATTATG

GCAGTTGGTGATTGTGCACGTTTTCATAGCCAGCTGTATGATCGTTGGGTTCGTATTGAA
AGCGTTCCGAATGCACTGGAACAGGCACGTAAAATTGCAGCAATTCTGTGTGGTAAAGTT
CCGCGTGATGAAGCAGCACCGTGGTTTTGGAGCGATCAGTATGAAATTGGTCTGAAAATG
GTTGGTCTGAGCGAAGGTTATGATCGCATTATTGTTCGTGGTAGCCTGGCACAGCCGGAT
TTTTCAGTTTTTTATCTGCAGGGTGATCGTGTGCTGGCAGTTGATACCGTTAATCGTCCG
GTTGAATTTAACCAGAGCAAACAAATTATCACCGATCGTCTGCCGGTGGAACCGAATCTG
CTGGGAGATGAAAGCGTGCCGCTGAAAGAAATTATTGCAGCAGCAAAAGCAGAACTGAGC
AGCGCATAA

Die Expression des erfindungsgemäßen Cytochroms P450 und etwaigen Ferredoxinen und Ferredoxin-Reduktasen in Bakterien, insbesondere in *E. coli,* ist besonders vorteilhaft unter Verwendung von Nukleinsäuren mit den Nukleinsäuresequenzen SEQ ID Nr. 5, SEQ ID Nr. 6, SEQ ID Nr. 11 und/oder SEQ ID Nr. 12. Daher betreffen weitere Aspekte der vorliegenden Erfindung eine Nukleinsäure (DNA und/oder RNA) mit einer Nukleinsäuresequenz ausgewählt aus der Gruppe bestehend aus SEQ ID Nr. 5, SEQ ID Nr. 6, SEQ ID Nr. 11 und SEQ ID Nr. 12 und Vektoren und/oder Zellen, insbesondere *E. coli* Zellen, umfassend mindestens eine dieser Sequenzen.

Es hat sich gezeigt, dass es besonders vorteilhaft ist, wenn die oben genannten Ferredoxine und Ferredoxin-Reduktasen gemeinsam mit Cytochrom P450 in einem Produktionsstamm (z.B. *E. coli* Stamm) gemeinsam exprimiert (coexprimiert) werden. Durch die Co-Expression der drei Proteine bzw. Enzyme, idealerweise unter demselben Promoter, kann ein ideales Gleichgewicht zwischen den Enzymen hergestellt werden, welches sich besonders vorteilhaft auf die enzymatische Umsetzung eines Substrats auswirkt.

Gemäß einer bevorzugten Ausführungsform der vorliegenden Erfindung ist die mindestens eine Oxidoreduktase ausgewählt aus der Gruppe bestehend aus Oxidoreduktase (EC: 1.1.1), Aldehyd Dehydrogenase (EC: 1.2.1), Aminosäuren Dehydrogenase (EC: 1.4.1), Flavin Reduktase (EC:1.5.1), Transhydrogenase (EC: 1.6.1), Nitrit Reduktase (EC: 1.7.1) und Phosphonat Dehydrogenase (EC: 1.20.1) ist, vorzugsweise ausgewählt aus der Gruppe bestehend aus Alkohol-Dehydrogenase, Hydroxysteroid-Dehydrogenase, Phosphit-Dehydrogenase und Zucker-Dehydrogenase

Um den im erfindungsgemäßen Verfahren eingesetzten Redoxpartnersystems, insbesondere den dabei eingesetzten Kofaktor (NADH/NAD⁺ und/oder NADPH/NADP⁺), während der Umsetzung des Steroids mit der allgemeinen Formel (I) in ein 7-Desoxysteroid mit der allgemeinen Formel (II) zu regenerieren, so dass die Umsetzungsreaktion in Richtung Produkt gedrängt wird, ist es von Vorteil ein System zur Regenerierung des Redoxpartnersystems, vorteilhafterweise Oxidoreduktasen, dem Reaktionsgemisch zuzusetzen. Oxidoreduktasen setzen Substrate durch Reduktion und Oxidation um, wobei im Zuge dieser Reaktionen NADH zu NAD⁺ bzw. NADPH zu NADP⁺ oxidiert bzw. NAD⁺ zu NADH bzw. NADP⁺ zu NADPH reduziert wird. Daher umfasst das System zur Regenerierung des Redoxpartnersystems vorzugsweise mindestens eine Oxidoreduktase und mindestens ein Substrat der mindestens einen Oxidoreduktase.

Die im erfindungsgemäßen Verfahren verwendete Oxidoreduktase ist vorzugsweise eine Alkohol- und/oder Zucker-Dehydrogenasen.

Gemäß einer weiteren bevorzugten Ausführungsform der vorliegenden Erfindung ist die Oxidoreduktase ausgewählt aus der Gruppe bestehend aus Glucose-Dehydrogenase, Glucose-6-Phosphat-Dehydrogenase, Arabinose-Dehydrogenase, Xylose-Dehydrogenase, Sorbitol-Dehydrogenase, Xylitol-Dehydrogenase, 12α-Hydroxysteroid-Dehydrogenase, 7α-Hydroxysteroid-Dehydrogenase, 20α-Hydroxysteroid Dehydrogenase, 17ß-Hydroxysteroid Dehydrogenase, 17α-Hydroxysteroid Dehydrogenase, 3α-Hydroxysteroid Dehydrogenase, 3ß-Hydroxy-delta5 Dehydrogenase, 11β-Hydroxysteroid Dehydrogenase und Formiat-Dehydrogenase.

Die Verwendung von einer oder mehreren der genannten Oxidoreduktasen ist besonders vorteilhaft, um die in der Umsetzungsreaktion eingesetzten Cofaktoren zu recyceln.

Es hat sich gezeigt, dass es besonders vorteilhaft ist Arabinose-Dehydrogenase, Sorbitol-Dehydrogenase und/oder Xylitol-Dehydrogenase zum Reaktionsgemisch zu geben, um einen hohen Umsatz des Substrats zum Produkt zu erzielen.

Das Reaktionsgemisch kann mindestens eine Oxidoreduktase und eine Hydroxylase umfassen. Besonders vorteilhaft ist die Zugabe einer Kombination von zwei oder drei oder weiterer Oxidoreduktasen zum Reaktionsgemisch, wobei eine Kombination einer 12α-Hydroxysteroid Dehydrogenase und einer 7α-Hydroxysteroid Dehydrogenase bzw. einer 12α-Hydroxysteroid Dehydrogenase, einer 7α-Hydroxysteroid Dehydrogenase und einer NAD(P)H-Oxidase bzw. einer NADH abhängigen Alkohol Dehydrogenase und einer Hydroxylase bzw. einer NADPH abhängigen Alkohol Dehydrogenase und einer Hydroxylase sich besonders für die Substratgemische eignet, beispielsweise für die gleichzeitige Oxidation und Hydroxylierung von natürlich vorkommenden Gemischen von Cholsäuren.

Um die Oxidations- bzw. Reduktionsreaktionen der Cofaktoren im Reaktionsgemisch des erfindungsgemäßen Verfahrens zu katalysieren, ist es notwendig mindestens ein Substrat für die darin vorhandenen Oxidoreduktasen bereitzustellen. Daher umfasst das Reaktionsgemisch mindestens ein Substrat der mindestens einen Oxidoreduktase ausgewählt aus der Gruppe bestehend aus Arabinose, Xylose, Glucose,Sorbitol, Xylitol, Cholan-24-Säure, 3α,12α-Dihydroxycholan-24-Säure 2,3-Butandiol, Acetoin, 2-Propanol, Glutamate, Ethanol, Phosphonate, Phosphite, Nitrite, 4-Methyl-2-pentanol, 2-butanol, 2-octanol, Cyclohexanol, Ethandiol, 1,2-Propandiol, 1-propanol, 1-butanol,3-hydroxy-butanoate und Formiat,Gemäß einer bevorzugten Ausführungsform der vorliegenden Erfindung wird das erfindungsgemäße Verfahren bei einer Temperatur von 10°C bis 40°C, vorzugsweise von 15°C bis 38°C, noch mehr bevorzugt von 20°C bis 30°C, noch mehr bevorzugt von 22°C bis 26°C, durchgeführt. Es hat sich erfindungsgemäß gezeigt, dass die Enzymaktivität von Cytochrom P450 für die erfindungsgemäße Reaktion in diesem Bereich besonders hoch ist.

Gemäß einer weiteren bevorzugten Ausführungsform der vorliegenden Erfindung wird das erfindungsgemäße Verfahren bei einem pH Wert von 6,5 bis 8,5, vorzugsweise von 7 bis 8, noch mehr bevorzugt von 7,2 bis 7,8, durchgeführt. Die Enzymaktivität von Cytochrom P450 ist bei diesem pH Wert am höchsten, um einen entsprechenden Umsatz des Substrats zu gewähren.

Die Hydroxylierung von Desoxysteroiden bzw. Desoxysteroidderivaten kann regioselektiv an der Position 7 des Steroid-Grundgerüsts erfolgten. Dadurch kann insbesondere stereoselektiv eine 7beta-Hydroxylgruppe eingeführt werden, so dass z.B. Ursocholsäure und/oder Ursocholsäurederivate hergestellt werden können.

Bei dem Verfahren gemäß der vorliegenden Erfindung kann die Isolierung des Produkts auf unterschiedliche Art und Weise erfolgen. Beispielsweise kann das Produkt durch ein geeignetes organisches Lösungsmittel aus der Reaktionsmischung extrahiert werden. Je nach Substrat sind solche Lösungsmittel in der Literatur beschrieben. Cholsäuren und deren Derivate gemäß der vorliegenden Erfindung können aus Reaktionsmischungen zum Beispiel mit Ethylacetat isoliert werden, ggf. nach Ansäuern der Reaktionsmischung, z.B. mit HCl. Einen speziellen Fall stellt ein Verfahren dar, bei dem Gallensäuren in Form eines Salzes, z.B. eines Natriumsalzes in wässriger Lösung vorliegen. In diesem Fall kann eine Fällung des Produkts durch Ansäuern der Reaktionsmischung erfolgen. Dazu kann der Reaktionsmischung, z.B. HCl oder verdünnte HCl in ausreichender Menge zugesetzt werden. Wird dabei ein pH-Wert von zum Beispiel 1 bis 4, vorzugsweise von 2 bis 3 erreicht, liegt das Produkt überwiegend in Form einer Suspension vor. Das Produkt kann dann durch gängige Methoden, wie z.B. Filtration oder Zentrifugation aus der Reaktionsmischung entfernt werden. Eine weitere Alternative, die beispielsweise zur Produktisolierung verwendet werden kann sind chromatographische Methoden, wie z.B. Affinitätschromatographie oder Ionenaustauscher-Chromatographie. Weiterhin ist beispielsweise die Gewinnung von Produkt durch die Verdampfung des Reaktions-Lösungsmittels möglich.

Alternativ kann bei einem Verfahren gemäß der vorliegenden Erfindung das/die Produkt(e) nach der Reaktion auch in der Reaktionsmischung verbleiben, z.B. um noch weitere Reaktionen durchzuführen und ggf. nach Anschluss dieser Reaktionen ein Endprodukt zu isolieren. Ebenso ist es denkbar, dass das Substrat/die Substrate für das Verfahren gemäß vorliegender Erfindung durch vorangegangene oder parallel ablaufende Reaktionen im selben Reaktionsansatz erzeugt werden.

Ein weiterer Aspekt der vorliegenden Erfindung betrifft ein Nukleinsäurekonstrukt umfassend ein Nukleinsäuremolekül kodierend für ein Cytochrom P450-Enzym wie oben definiert an dessen 3'-Ende und/oder 5'-Ende direkt oder über einen Spacer mindestens ein Nukleinsäuremolekül kodierend für ein Polypeptid ausgewählt aus der Gruppe bestehend aus einem Ferredoxin, einer Ferredoxin-Reduktase und einer Oxidoreduktase gebunden ist.

Die erfindungsgemäßen Nukleinsäurekonstrukte sind besonders geeignet zur Herstellung eines Steroids wie eingangs definiert. Durch die Expression des Cytochrom P450-Enzyms und mindestens eines Proteins ausgewählt aus der Gruppe bestehend aus Ferredoxinen, Ferredoxin-Reduktasen und Oxidoreduktasen ausgehend von einem Nukleinsäurekonstrukt wird es ermöglicht, diese Enzyme bzw. Proteine in einer Menge zu produzieren, die für eine effiziente Durchführung des erfindungsgemäßen Verfahren nötig sind. Insbesondere von Vorteil ist es, wenn sämtliche dieser Proteine unter der Kontrolle desselben Promoters am erfindungsgemäßen Nukleinsäurekonstrukt exprimiert werden. Ein weiterer Aspekt der vorliegenden Erfindung Vektor umfassend ein Nukleinsäurekonstrukt gemäß der vorliegenden Erfindung.

Das Nukleinsäuremolekül kodierend für ein Cytochrom P450-Enzym kann entweder direkt oder über einen Spacer bzw. eine Spacer-Sequenz an weitere Nukleinsäuremoleküle gebunden sein, die für Enzyme bzw. Proteine kodieren, die im erfindungsgemäßen Verfahren eingesetzt werden können. Der Vorteil eines derartigen Konstrukts liegt darin, dass ein solches Konstrukt es ermöglicht, die im erfindungsgemäßen Verfahren eingesetzten Enzyme und Proteine, insbesondere Cytochrom P450 und Ferredoxin und/oder Ferredoxin-Reduktase, in einer vergleichbaren Menge zu exprimieren.

"Spacer" bzw. "Spacer-Sequenz", wie hier verwendet, ist eine Nukleinsäuresequenz die weder ein Stop-Codon noch ein sonstiges funktionelles Motiv aufweist. Ein Spacer bzw. eine Spacer-Sequenz dient als Abstandshalter zwischen zwei ORF, um gegebenenfalls die Transkription dieser ORFs zu verbessern.

Gemäß einer bevorzugten Ausführungsform der vorliegenden Erfindung ist das Cytochrom P450 Enzym durch eine Nukleinsäure kodiert, welche mindestens 80%, vorzugsweise mindestens 90%, insbesondere 100%, ident ist mit der Nukleinsäuresequenz SEQ ID Nr. 3, SEQ ID Nr. 4, SEQ ID Nr. 5 oder SEQ ID Nr. 6.

Gemäß einer weiteren bevorzugten Ausführungsform der vorliegenden Erfindung umfasst das Ferredoxin die Aminosäuresequenz SEQ ID Nr. 7.

Gemäß einer besonders bevorzugten Ausführungsform der vorliegenden Erfindung ist die Ferredoxin-Reduktase eine Adrenodoxin-Reduktase, vorzugsweise eine Putidaredoxin-Reduktase.

Die Ferredoxin-Reduktase umfasst vorzugsweise die Aminosäuresequenz SEQ ID Nr. 8.

Ein weiterer Aspekt der vorliegenden Erfindung betrifft einen Vektor umfassend ein Nukleinsäurekonstrukt gemäß der vorliegenden Erfindung.

Der erfindungsgemäße Vektor kann ein Klonierungs- oder Expressionsvektor sein und je nach Organismus, in dem dieser eingebracht wird, entsprechende Abschnitte aufweisen, um beispielsweise die Transkription eines ORFs zu ermöglichen.

Ein noch weiterer Aspekt der vorliegenden Erfindung betrifft eine Wirtszelle umfassend ein Nukleinsäurekonstrukt gemäß der vorliegenden Erfindung.

Die erfindungsgemäße Wirtszelle kann zur Klonierung oder zur Expression der rekombinant eingebrachten auf einem Nukleinsäurekonstrukt befindlichen ORFs verwendet werden.

Das Lysat einer solchen Wirtszelle, sofern diese mindestens eines der im erfindungsgemäßen Verfahren benötigten Enzyme bzw. Proteine intra- oder extrazellulär exprimiert hat, kann im erfindungsgemäßen Verfahren eingesetzt werden. Das 7-Desoxy-Steroid oder ein Derivat davon mit der allgemeinen Formel (II) wird dabei vorzugsweise mit einem Kulturüberstand und/oder einem Lysat einer Wirtszelle gemäß der vorliegenden Erfindung in Kontakt gebracht wird. Somit wird gemäß einer bevorzugten Ausführungsform der vorliegenden Erfindung 7-Desoxy-Steroid oder ein Derivat davon mit der allgemeinen Formel (II) mit mindestens einem Kulturüberstand und/oder einem Lysat mindestens einer Wirtszelle, welche in der Lage ist, mindestens ein Ferredoxin, mindestens eine Ferredoxin-Reduktase und/oder mindestens eine Oxidoreduktase zu exprimieren, in Kontakt gebracht.

### BEISPIELE

Die vorliegende Erfindung wird anhand der folgenden Beispiele eingehender dargelegt, ohne jedoch darauf beschränkt zu sein.

### Beispiel 1: Test von Bakterienstämmen

Die folgenden bakteriellen Stämme wurden bei der Deutschen Stammsammlung für Mikroorganismen und Zellkulturen (DSMZ) bezogen: *Saccharothrix longispora* (DSM-43749), *Catellatospora citrae* (DSM-44097), *Streptomyces hygroscopicus* subsp. *hygroscopicus* (DSM-40578) und *Asanoa ferruginea* (DSM-44099). Die Stämme wurden unter Standardbedingungen kultiviert, wie von DSMZ empfohlen. Sobald das Wachstum der Kulturen zu einer sichtbaren Trübung geführt hatte, wurde Desoxycholsäure (0,5 mM) zugegeben und für bis zu 72 h weiter kultiviert. Überstände der Kulturen wurden nach einem Zentrifugationsschritt mit Ethylacetat extrahiert und mittels HPLC und GC/MS analysiert. Im HPLC-Chromatogramm der Reaktion mit *Streptomyces hygroscopicus* wurde ein Peak registriert, dessen Retentionszeit mit der von Ursocholsäure übereinstimmt. Die GC/MS-Analyse ergab einen Hinweis darauf, dass der potentielle Ursocholsäure-Peak von einer Gallensäure mit 3 Hydroxylgruppen stammt. Die Untersuchung der anderen Stämme ergab keinen Hinweis auf 7-hydroxylierte Produkte von Desoxycholsäure.

### Beispiel 2: Genomsequenzierung und Annotierung der P450-Gene

Nach Kultivierung von *Streptomyces hygroscopicus* subsp. *hygroscopicus* (DSM-40578) entsprechend der Vorschrift von DSMZ wurde die genomische DNA des Stamms isoliert (Kieser et al. (2000), Practical Streptomyces genetics (Norwich: John Innes Foundation)). Das Genom wurde mittels Illumina MiSeq sequenziert und ein Assembly basierend auf dem bekannten Genom von *Streptomyces rapamycinicus* durchgeführt (Microsynth GmbH, Switzerland). Durch Homologie-Vergleiche konnten 42 P450-Gene identifiziert werden.

### Beispiel 3: Klonierung Expressions-System

Unter Verwendung des Restriktionsenzyms XhoI wurde folgendes Konstrukt umfassend kodierende Regionen für Putidaredoxin-Reduktase (PtR) und Putidaredoxin (Ptx) in das Plasmid pJ411 (DNA 2.0) kloniert.

Synthetische DNA (Life Technologies): 5', XhoI-Schnittstelle, HindIII-Schnittstelle, ca. 50 bp spacer-DNA, ribosome binding site (rbs), ORF (open reading frame; offener Leserahmen) Putidaredoxin-Reduktase (PtR), ca. 50 bp spacer-DNA, rbs, ORF Putidaredoxin (Ptx), XhoI-Schnittstelle, 3'.

Das Ergebnis des Klonierungs-Schritts wurde mittels Restriktionsenzym-Verdau und DNA-Sequenzierung überprüft.

Anschließend wurde unter Verwendung der Restriktionsenzyme NdeI und HindIII jeweils ein ORF kodierend für die P450-Enzyme, die in Beispiel 2 identifiziert wurden, in die oben erwähnte synthetische DNA bzw. Plasmid (Life Technologies) kloniert. Das Ergebnis wurde wieder mit Restriktionsenzym-Verdau und DNA-Sequenzierung verifiziert. Der in diesem Beispiel verwendete Expressionsvektor sowie die verwendeten Redoxpartner stellen nur eine beispielhaft ausgewählte Möglichkeit dar, die erfindungsgemäßen Cytochrom P450 Enzyme zu exprimieren.

Die mit den identifizierten P450-Kandidaten (s. Beispiel 2) hergestellten Expressionsplasmide, können zur gemeinsamen Expression der jeweiligen P450 Proteine gemeinsam mit Putidaredoxin-Reduktase und Putidaredoxin verwendet werden. Die 3 ORFs der jeweiligen Expressionsplasmide werden unter Kontrolle eines T7-Promotors auf einer gemeinsamen mRNA aber als getrennte Polypeptide exprimiert.

### Beispiel 4: Expression P450/Ptx/PtR

Nach der Genom-Sequenzierung von *Streptomyces hygroscopicus* subsp. *hygroscopicus* lagen 42 P450-Sequenzen vor, die als Kandidaten für eine mögliche Desoxycholsäure-7-Hydroxylase in Frage kamen. Zur Identifizierung des gesuchten Enzyms wurden ORFs der Kandidaten in das in Beispiel 3 beschriebene Expressions-System und in einen pJ411 (DNA 2.0) Expressionsvektor ohne kodierende Regionen für Putidaredoxin-Reduktase (PtR) und Putidaredoxin (Ptx) kloniert. Zur Expression wurde folgendes Protokoll verwendet.

### TB-P450-Expressionsmedium:

Terrific broth (TB) Medium
+ 50 µg/ml Kanamycin
+ 0.5 mM 5-Aminolävulinsäure (von 100x Stammlösung)
+ 1 mM Thiamin (von 100x Stammlösung)
+ 1 mM MgCl₂ + 2.5 mM Ammoniumsulfat + 50 µM FeCl₃ (von 100x Stammlösung)
+ 0.5 mM IPTG (von 1 M Stammlösung)
   (die Zusätze wurden jeweils 0.2 µm sterilfiltriert)

### P450 Lysepuffer:

100 mM Tris pH 7,5
20 % (v/v) Glycerin
1 mg/ml Lysozym

Die zu testenden Konstrukte der P450-Kandidaten wurden in den *E. coli* Expressionsstamm BL21 (DE3) transformiert. Von Einzelkolonien wurden Übernachtkulturen angeimpft (LB (lysogeny broth) + Kanamycin). Am nächsten Tag wurden damit 1:100 Expressionskulturen angeimpft (150 ml TB (terrific broth)-P450-Expressionsmedium) und in Schikanenkolben (1 L) zunächst bei 37°C für 3 h geschüttelt. Anschließend wurde die Temperatur auf 24°C gesenkt und 22 h weitergeschüttelt. Die Kulturen wurden durch Zentrifugation bei 5000 g für 10 min geerntet, 1x mit 0.9 % (w/v) NaCl gewaschen und Pellets bei -80°C eingefroren. Die Zellpellets wurden aufgetaut, gewogen und mit einer äquivalenten Menge P450-Lysepuffer resuspendiert, 1 h auf Eis inkubiert und anschließend mittels Sonifier aufgeschlossen. Nach Zentrifugation (30 min, 21000 g) wurden die Überstände für Test-Reaktionen verwendet.

### Beispiel 5: Test von P450-Kandidaten auf DA-Hydroxylierung

### Reaktions-Mischung:

10-80 µl 100 mM NADH (Redoxcofaktor)
250 µl 1 M Tris-HCl pH 7,5
17,5 µl Glycerin (50%)
100 µl 50 mM Desoxycholsäure-Lösung pH 8,5 (final 10 mM)
50 µl *E. coli* Lysat P450/PtR/Ptx (s. Beispiel 4)
17,5-87,5 µl dH₂O

Die Reaktionen wurden in 1,5 ml Schraubflaschen angesetzt und mit Deckel mit Alufolie verschlossen. Die Folie wurde an mehreren Stellen eingestochen. Es wurde für 18 h bei 24°C leicht geschüttelt. 200 µl des Reaktionsansatzes wurden mit 600 µl Acetonitril / 5 µl H₃PO₄ (50%) verdünnt und 15 Minuten bei 55 °C inkubiert. Anschließend wurden die Proben 5 Minuten bei 20817 rcf zentrifugiert und mittels HPLC/DAD analysiert (z.B. Agilent 1200 Series; Säule: Merck Purospher STAR RP-18e 125 x 4 mm, 5 µm; Flussrate: 1,5 ml/min, Gradient H₂O+H₃PO₄ (pH=2.6) / Acetonitril). Einer der untersuchten Kandidaten ("P450_c866") war in der Lage, Desoxycholsäure zu Ursocholsäure zu hydroxylieren. Dabei wurde die eingesetzte Desoxycholsäure umgesetzt (s. folgende Tabelle). Die Identität des Produkts Ursocholsäure wurde durch GC/MS-Analyse sowie durch 2D-NMR verifiziert.

| Redoxcofaktor [µl] | Ursocholsäure [µg / ml] | Umsatz [%] |
|---|---|---|
| 10 | 30 | 3,4 |
| 30 | 48 | 5,3 |
| 50 | 57 | 6,2 |
| 80 | 110 | 11,6 |

In diesem Beispiel wird ein Redoxcofaktor (NADH) durch die P450/Ptx/PtR Reaktion oxidiert.

### Beispiel 6: Test von P450-Kandidaten auf DA-Hydroxylierung mit Cofaktor-Recycling Arabinose-Dehydrogenase

Reaktions-Mischung:
65 µl 100 mM NADH (final 0.5 mM)
1 ml 1 M Tris-HCl pH 7.5 + 20 % (v/v) Glycerin
130 µl 50 mM Desoxycholsäure-Lösung pH 8.5 (final 0.5 mM)
6.5 µl Chloramphenicol-Lösung (final 20 µg/ml)
100 µl L-Arabinose-Dehydrogenase
(aus *Burkholderia vietnamiensis,* rekombinant in *E*. *coli* exprimiert, 400 U/ml)
98 mg L-Arabinose (final 50 mM)
2.0 ml *E. coli* Lysat P450/PtR/Ptx (s. Beispiel 4)
9.6 ml dH₂O

Die Reaktionen wurden in 50 ml Erlenmeyerkolben ohne Schikane angesetzt und mit Alufolie verschlossen. Die Folie wurde an mehreren Stellen eingestochen. Es wurde für 16 h bei 24°C leicht geschüttelt. Die im Überstand vorhandenen Substanzen wurden mit Ethylacetat extrahiert und eingedampft. Es wurde in einem geringeren Volumen HPLC-Laufmittel gelöst (Methanol/Acetonitril/H₂O+H₃PO₄ (pH=3.0); 40:30:33). Anschließend wurden die Proben mittels HPLC/RID analysiert (z.B. Agilent 1200 Series; Säule: Agilent ZORBAX Eclipse XDB-C18 4.6 x 150 mm, 5 µm; Flussrate: 0.8 ml/min). Einer der untersuchten Kandidaten ("P450_c866") war in der Lage, Desoxycholsäure zu Ursocholsäure zu hydroxylieren. Dabei wurde die eingesetzte Desoxycholsäure nahezu vollständig umgesetzt (>95 %). Die Identität des Produkts Ursocholsäure wurde durch GC/MS-Analyse sowie durch 2D-NMR verifiziert (Daten nicht gezeigt).

In diesem Beispiel wird ein Redoxcofaktor (NADH) durch die P450/Ptx/PtR Reaktion oxidiert. Durch die Cofaktor-Regenerierung (hier beispielhaft mittels der Zucker-Dehydrogenase Arabinose-Dehydrogenase) wird der Redoxcofaktor wieder zu seinem ursprünglichen Zustand reduziert (wobei hier Arabinose zu Arabinolacton/Arabonsäure oxidiert wird). Dadurch wird der Einsatz substöchiometrischer Menge von Redoxcofaktor ermöglicht.

### Beispiel 7: Beispiele: Umsatz abhängig vom Cofaktorkonzentration Umsatz mit Cofaktorrecycling

### Reaktions-Mischung:

10 µl 10 mM NAD+
250 µl 100 mM TEA pH 8,2
25 µl Glycerin (50%)
100 µl 50 mM Desoxycholsäure-Lösung pH 8.0 (final 10 mM)
6,75 mg Zellen (wW) als 22,5 % Suspension in 100 mM TEA pH 8.0 und 25 % Glycerin
5 µl Katalase (bovine, Sigma 4 mg/ml)
1,7 Einheiten Xylitol/Sorbitol Dehydrogenase
25 µl 2M Sorbitol (final 100 mM)
70,8 µl dH₂O

Die Reaktionen wurden in 1,5 ml Schraubflaschen angesetzt und mit Deckel mit Alufolie verschlossen. Die Folie wurde an mehreren Stellen eingestochen. Es wurde für 18 h bei 24°C leicht geschüttelt.

Die Rückgewinnung von NADH erfolgte durch Sorbitol-, bzw. Xylitol Dehydrogenase in Gegenwart von Sorbitol und NAD⁺.

200 µl des Reaktionsansatzes wurden mit 600 µl Acetonitril / 5 µl H₃PO₄ (50%) verdünnt und 15 Minuten bei 55 °C inkubiert. Anschließend wurden die Proben 5 Minuten bei 20817 rcf zentrifugiert und mittels HPLC/DAD analysiert (z.B. Agilent 1200 Series; Säule: Merck Purospher STAR RP-18e 125 x 4 mm oder Agilent Zorbax XDB-C8 mm 150 x 4,6 mm, 3,5 µm, 5 µm; Flussrate: 1,5 ml/min, Gradient H₂O+H₃PO₄ (pH=2.6) / Acetonitril).

Unter den oben genannten Bedingungen wurde die eingesetzte Desoxycholsäure quantitativ (100% Umsatz) zu Ursocholsäure umgesetzt. Die Identität des Produkts Ursocholsäure wurde durch GC/MS-Analyse sowie durch 2D-NMR verifiziert.

In diesem Beispiel wird ein durch das Cofaktorrecyclingsystem Sorbitol/Xylitol Dehydrogenase/Sorbitol/NAD⁺ gewonnener Redoxcofaktor (NADH) für die Hydroxylierungsreaktion verwendet. Es können jedoch auch andere Systeme zum Cofaktorrecycling verwendet werden (siehe folgende Tabelle) .

| Regenerationsenzym/Oxidoredukatse | µl Lysat Oxidoreduktase | Redoxcofaktor | Konzentration [mM] | Substrat Oxidoreduktase | Konzentration [mM] | DOCA-Umsatz [%] |
|---|---|---|---|---|---|---|
| Xylitol/Sorbitol Dehydrogenase | 1,7 | NAD⁺ | 0,2 | Sorbitol | 100 | 100% |
| Arabinose-Dehydrogenase | 3 | NADH | 0,5 | Arabinose | 50 | 95% |
| 12α-Hydroxysteroid Dehydrogenase | 4 | NAD⁺ | 0,77 | Cholsäure^{[1]} | 49 | 28,5 |
| 7α-Hydroxysteroid Dehydrogenase | 4 | NAD⁺ | 0,77 | Cholsäure^{[1]} | 49 | 17,2% |
| 12α-Hydroxysteroid Dehydrogenase / 7α-Hydroxysteroid Dehydrogenase | 5 / 2,3 | NAD⁺ | 0,28 | Cholsäure^{[2]} | 51,5 | 91% |
| 12α-Hydroxysteroid Dehydrogenase / 7α-Hydroxysteroid Dehydrogenase / Oxidase | 5 / 2,3 /1,3 | NAD⁺ | 0,28 | Cholsäure^{[2]} | 51,5 | 100% |
| NADH abhängige Alkohol Dehydrogenase / 6,75 mg Hydroxylase | 6 | NAD⁺ | 0,19 | 2,3-Butandiol | 427 | 98% |
| NADPH abhängige Alkohol Dehydrogenase / 6,75 mg Hydroxylase | 2 | NADP+ | 0,19 | 2-Propanol | 909 | 100% |
| Formiat Dehydrogenase | 4 | NAD⁺ | 0,82 | Na-Formiat | 204 | 21,5% |

| | | | | | | |
|---|---|---|---|---|---|---|
| [1] Eingesetzt als 20% Cholsäure Lösung [2] Eingesetzt als Gallensäuren Lösung (237 mM Cholsäure; 45 mM Desoxycholsäure) | | | | | | |

### Beispiel 8: Quantitativer LCA (Lithocholsäure) Umsatz

### Reaktions-Mischung:

10 µl 10 mM NAD+
250 µl 100 mM TEA pH 8,2
10 mM (final)Lithocholsäure
9,2 mg Zellen (wW) als 22,5 % Suspension in 100 mM TEA pH 8.0 und 25 % Glycerin
5 µl Katalase (bovine, Sigma 4 mg/ml)
1,7 Units Xylitol/Sorbitol Dehydrogenase
25 µl 2M Sorbitol (final 100 mM)
176 µl dH₂O

Die Reaktionen wurden in 1,5 ml Schraubflaschen angesetzt und mit Deckel mit Alufolie verschlossen. Die Folie wurde an mehreren Stellen eingestochen. Es wurde für 18 h bei 24°C leicht geschüttelt.

Die Rückgewinnung von NADH erfolgte durch die Sorbitol-, bzw. Xylitol Dehydrogenase in Gegenwart von Sorbitol und NAD⁺.

200 µl des Reaktionsansatzes wurden mit 600 µl Acetonitril / 5 µl H₃PO₄ (50%) verdünnt und 15 Minuten bei 55 °C inkubiert. Anschließend wurden die Proben 5 Minuten bei 20817 rcf zentrifugiert und mittels HPLC/DAD analysiert (z.B. Agilent 1200 Series; Säule: Merck Purospher STAR RP-18e 125 x 4 mm oder Agilent Zorbax XDB-C8 mm 150 x 4,6 mm, 3,5 µm, 5 µm; Flussrate: 1,5 ml/min, Gradient H₂O+H₃PO₄ (pH=2.6) / Acetonitril).

Unter den oben genannten Bedingungen wurde nach dem Umsatz ausschließlich Ursodeoxycholsäure detektiert.

## Patentansprüche

1. Verfahren zur Herstellung eines Steroids mit der allgemeinen Formel (I) 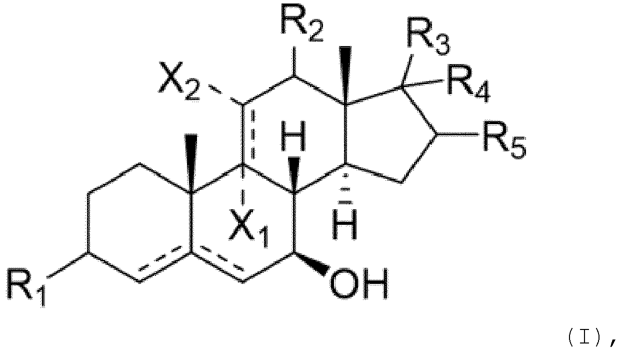 wobei
X₁ und X₂ unabhängig voneinander H, Cl, F, Br, I, CF₃, ein C₁ bis C₆ Alkylrest, OH, ein C₁ bis C₆ Alkoxyrest, CN, NO₂, N(R₆)₂, eine Epoxygruppe, CHO oder ein CO₂R₆-Rest sind, wobei
R₆ -C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph,-C(O)CH₂Ph ist,
R₁ und R₂ unabhängig voneinander H, OH, OR₇ oder O sind, wobei
R₇ -C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph,-C(O)CH₂Ph ist,
R₃ H, OH, OR₈, ein C₁ bis C₁₀ Alkylrest, ein C₁ bis C₁₀ Alkenylrest, -CHO, -C(O)(CH₃), -C(O)(CH₂OH),-CH(CH₃)C(O)CH₃, -CH (CH₃)((CH₂)₂CO₂R₉) oder-CH(CH₃)((CH₂)₂CONHR₉) ist, wobei
R₈-C (O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph, oder-C(O)CH₂Ph ist, und
R₉-CH₃, -CH₂COOH, -CH₂CH₃, -CH(CH₃)₂, -(CH₂)₂CH₃,-(CH₂)₂SO₃H, C(CH₃)₃, -(CH₂)₃CH₃, -CH(CH₃)CH₂CH₃,-CH₂CH₂(CH₃)₂, eine Arylgruppe oder eine Alkylaryl-gruppe ist,
R₄ H, OH, oder -OR₁₀ ist, wobei
R₁₀-C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph, oder-C(O)CH₂Ph ist und
R₅ H, CF₃, ein C₁ bis C₆ Alkylrest, ein C₁ bis C₆ Alkenyl-rest, OH, O, oder ein C₁ bis C₆ Alkoxyrest ist, wobei
die gestrichelte Linie eine optionale Doppelbindung bedeutet, mit der Maßgabe, dass der B-Ring keine Doppelbindung aufweist, wenn der A-Ring eine C4-C5-Doppelbindung und der C-Ring keine Doppelbindung aufweist, wenn X₁ und X₂ eine Epoxygruppe ausbilden,
umfassend den Schritt des Umsetzens eines 7-Desoxysteroids mit der allgemeinen Formel (II) 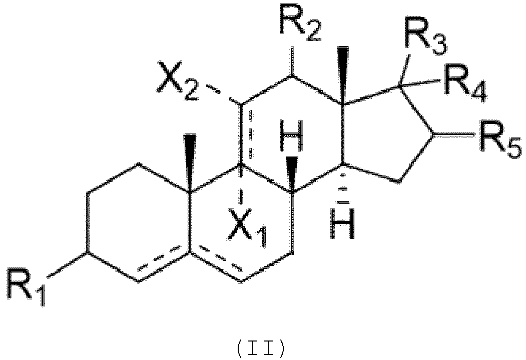 mit einer Cytochrom P450-Hydroxylase oder einer funktionellen Variante davon in Anwesenheit mindestens eines Redoxpartnersystems und eines Systems zur Regenerierung des Redoxpartnersystems.

2. Verfahren nach Anspruch 1, **dadurch gekennzeichnet, dass** X₁, X₂, R₄ und R₅ H sind und
R₁ und R₂ unabhängig voneinander H, OH, OR₈ oder O ist, wobei
R₈-C(O)H, -C(O)CH₃, -C(O)CH₂CH₃, -C(O)(CH₂)₂CH₃,-C(O)CH(CH₃)₂, -C(O)(CH₂)₃CH₃, -C(O)CH(CH₃)CH₂CH₃,-C(O)CH₂CH₂(CH₃)₂, -C(O)C(CH₃)₃, -C(O)Ph, -C(O)CH₂Ph ist,
R₃ ein C₁ bis C₁₀ Alkylrest, ein C₁ bis C₁₀ Alkylenrest,-CH(CH₃)((CH₂)₂CO₂R₉) oder -CH (CH₃)((CH₂)₂CONHR₉) , wobei
R₉-CH₃, -CH₂COOH, -CH₂CH₃, -CH(CH₃)₂, -(CH₂)₂CH₃,-(CH₂)₂SO₃H, C(CH₃)₃, -(CH₂)₃CH₃, -CH(CH₃)CH₂CH₃,-CH₂CH₂(CH₃)₂, eine Arylgruppe oder eine Alkylarylgruppe ist.

3. Verfahren nach Anspruch 1 oder 2, **dadurch gekennzeichnet, dass** die Arylgruppe ausgewählt ist aus der Gruppe bestehend aus einem Phenylrest, einem mit F, Cl, Br, NO₂ oder CH₃ substituierten Phenylrest und einem Heteroaryl.

4. Verfahren nach einem der Ansprüche 1 bis 3, **dadurch gekennzeichnet, dass** die Alkylarylgruppe ausgewählt ist aus der Gruppe bestehend aus einer Benzylgruppe, einer halogenierten Benzylgruppe, wobei das Halogen F, Cl oder Br ist, und einer mit NO₂ substituierten Benzylgruppe.

5. Verfahren nach einem der Ansprüche 1 bis 4, **dadurch gekennzeichnet, dass** R₁CH(CH₃)((CH₂)₂CO₂R₅), R₂H, R₃O oder OH, R₄OH und R₅H ist.

6. Verfahren nach einem der Ansprüche 1 bis 5, **dadurch gekennzeichnet, dass** das Steroid mit der allgemeinen Formel (I) ausgewählt ist aus der Gruppe bestehend aus 7β,12α-Dihydroxy-3-keto-5β-cholan-24-säure, 3α,7β-Dihydroxy-12-keto-5β-cholan-24-säure, 7β-Hydroxy-3,12-diketo-5β-cholan-24-säure, 3α,7β-Dihydroxy-5β-cholan-24-säure und 3β,7β-Dihydroxy-5β-cholan-24-säure.

7. Verfahren nach einem der Ansprüche 1 bis 6, **dadurch gekennzeichnet, dass** das 7-Desoxy-Steroid ausgewählt ist aus der Gruppe bestehend aus 3α,12α-Dihydroxy-5β-cholan-24-Säure, 3α,12β-Dihydroxy-5β-cholan-24-Säure, 3β,12α-Dihydroxy-5β-cholan-24-Säure, 3β,12β-Dihydroxy-5β-cholan-24-Säure, 3β-Hydroxy-12-Keto-5β-cholan-24-Säure, 3-Keto,12β-hydroxy-5β-cholan-24-Säure, 3-Keto,12α-hydroxy-5β-cholan-24-Säure, 3α-Hydroxy-5β-cholan-24-Säure, 3-keto-5β-cholan-24-Säure, 3β-Hydroxy-5β-cholan-24-Säure und Ester der jeweiligen Säure.

8. Verfahren nach einem der Ansprüche 1 bis 7, **dadurch gekennzeichnet, dass** das Cytochrom P450-Enzym eine Aminosäuresequenz umfasst, die mindestens 80%, vorzugsweise mindestens 90%, insbesondere 100%, ident ist mit der Aminosäuresequenz SEQ ID Nr. 1 oder 2.

9. Verfahren nach einem der Ansprüche 1 bis 8, **dadurch gekennzeichnet, dass** das Cytochrom P450-Enzym durch eine Nukleinsäure kodiert ist, welche mindestens 80%, vorzugsweise mindestens 90%, insbesondere 100%, ident ist mit der Nukleinsäuresequenz SEQ ID Nr. 3, SEQ ID Nr. 4, SEQ ID Nr. 5 oder SEQ ID Nr. 6.

10. Verfahren nach einem der Ansprüche 1 bis 9, **dadurch gekennzeichnet, dass** das mindestens eine Redoxpartnersystem
(i) Ferredoxin, Ferredoxin-Reduktase und NAD(P)H;
(ii) Cytochrom P450 Reduktase und NAD(P)H; oder
(iii) NAD(P)H
umfasst.

11. Verfahren nach Anspruch 10, **dadurch gekennzeichnet, dass** das Ferredoxin ausgewählt ist aus der Gruppe bestehend aus Adrenodoxine, Putidaredoxine und Flavodoxine.

12. Verfahren nach Anspruch 10 oder 11, **dadurch gekennzeichnet, dass** die mindestens eine Ferredoxin-Reduktase ausgewählt ist aus der Gruppe der Flavodoxin-Reduktasen und Putidaredoxin-Reduktase.

13. Verfahren nach einem der Ansprüche 1 bis 12, **dadurch gekennzeichnet, dass** das System zur Regenerierung des Redoxpartnersystems mindestens eine Oxidoreduktase und mindestens ein Substrat der mindestens einen Oxidoreduktase umfasst.

14. Verfahren nach Anspruch 13, **dadurch gekennzeichnet, dass** die mindestens eine Oxidoreduktase ausgewählt ist aus der Gruppe bestehend aus Oxidoreduktase (EC: 1.1.1), Aldehyd Dehydrogenase (EC: 1.2.1), Aminosäuren Dehydrogenase (EC: 1.4.1), Flavin Reduktase (EC:1.5.1), Transhydrogenase (EC: 1.6.1), Nitrit Reduktase (EC: 1.7.1) und Phosphonat Dehydrogenase (EC: 1.20.1) ist, vorzugsweise ausgewählt aus der Gruppe bestehend aus Alkohol-Dehydrogenase, Hydroxysteroid-Dehydrogenase, Phosphit-Dehydrogenase und Zucker-Dehydrogenase.

15. Verfahren nach Anspruch 13 oder 14, **dadurch gekennzeichnet, dass** die mindestens eine Oxidoreduktase ausgewählt ist aus der Gruppe bestehend aus Glucose-Dehydrogenase, Glucose-6-PhosphatDehydrogenase, Arabinose-Dehydrogenase, Xylose-Dehydrogenase, Sorbitol-Dehydrogenase, Xylitol-Dehydrogenase, 12α-Hydroxysteroid-Dehydrogenase, 7α-Hydroxysteroid-Dehydrogenase, 20α-Hydroxysteroid Dehydrogenase, 17β-Hydroxysteroid Dehydrogenase, 17α-Hydroxysteroid Dehydrogenase, 3α-Hydroxysteroid Dehydrogenase, 3β-Hydroxy-delta5 Dehydrogenase, 11β-Hydroxysteroid Dehydrogenase und Formiat-Dehydrogenase.

16. Verfahren nach einem der Ansprüche 13 bis 15, **dadurch gekennzeichnet, dass** das mindestens eine Substrat der mindestens einen Oxidoreduktase ausgewählt ist aus der Gruppe bestehend aus Arabinose, Xylose, Glucose, Sorbitol, Xylitol, Cholan-24-Säure, 3α,12α-Dihydroxycholan-24-Säure 2,3-Butandiol, Acetoin, 2-Propanol, Glutamate, Ethanol, Phosphonate, Phosphite, Nitrite, 4-Methyl-2-pentanol, 2-butanol, 2-octanol, Cyclohexanol, Ethandiol, 1,2-Propandiol, 1-propanol, 1-butanol, und Formiat, 3-hydroxy-butanoate.


SEQUENCE LISTING

<110>  Annikki GmbH
       Pharmazell GmbH

<120>  VERFAHREN ZUR HYDROXYLIERUNG VON STEROIDEN

<130>  23658-EP

<160>  12

<170>  PatentIn version 3.5

<210>  1
<211>  397
<212>  PRT
<213>  Streptomyces hygroscopicus

<400>  1

Met Leu Thr Thr Ala Glu Thr Thr Ser Ile Ala Tyr Pro Phe Asn Thr
1               5                   10                  15

Ala Glu Gly Leu Ala Leu Ser Glu Arg Tyr Glu Glu Ala Arg Asn Arg
            20                  25                  30

Thr Gly Leu Leu Arg Val Arg Met Pro Tyr Gly Glu Pro Ala Trp Leu
        35                  40                  45

Val Thr Arg Tyr Ala Asp Ala Arg Leu Val Leu Gly Asp Arg Arg Phe
    50                  55                  60

Ser Arg Ala Glu Ala Leu His His Asp Glu Pro Arg Gln Ser Glu Gly
65                  70                  75                  80

Arg Arg Asp Ser Gly Ile Leu Thr Met Asp Pro Pro Asp His Thr Arg
                85                  90                  95

Leu Arg Thr Leu Val Ala Lys Ala Phe Thr Val His Gln Val Glu Lys
            100                 105                 110

Leu Arg Pro Trp Val Arg Gln Leu Thr His Asp Leu Leu Asp Asp Leu
        115                 120                 125

Glu Ala Ala Gly Pro Pro Ala Asp Leu Val Asp Arg Tyr Ala Leu Pro
    130                 135                 140

Ile Pro Val Gly Val Ile Cys Ala Met Leu Gly Val Pro Gln Glu Asp
145                 150                 155                 160

Arg Pro Lys Phe Arg Val Trp Ser Asp Ala Ala Leu Ser Thr Ser Ser
                165                 170                 175

Leu Ser Ala Glu Gln Phe Ala Arg Asn Thr Asp Glu Leu Arg Ala Tyr
            180                 185                 190

Met Ala Gly Leu Ile Glu Asp His Arg Arg Thr Pro Arg Asp Asp Ile
        195                 200                 205

Met Thr Ser Leu Ile Glu Ala Arg Asp Ala Gly Asp Arg Leu Ser Glu
    210                 215                 220

Leu Glu Leu Val Asp Leu Cys Val Gly Ile Leu Val Ala Gly His Glu
225                 230                 235                 240

Thr Thr Ala Thr Gln Ile Pro Asn Phe Val Leu Thr Leu Leu Glu His
                245                 250                 255

Pro Asp Gln Leu Arg Arg Leu Arg Glu Asp Pro Ala Leu Ile Gln Gly
            260                 265                 270

Ala Val Glu Glu Leu Leu Arg Phe Val Pro Leu Gly Val Gly Ala Ala
        275                 280                 285

Gln Ala Arg Tyr Ala Thr Glu Asp Ile Glu Val Gly Gly Thr Leu Val
    290                 295                 300

Arg Ser Gly Glu Pro Val Leu Val Ala Val Gly Ser Ala Asn Arg Asp
305                 310                 315                 320

Ala Leu Arg Phe Asp Glu Pro Gly Val Leu Asn Val Ala Arg Pro Thr
                325                 330                 335

Thr Gln His Leu Gly Phe Gly His Gly Val His His Cys Leu Gly Ala
            340                 345                 350

Pro Leu Ala Arg Leu Glu Leu Gln Glu Ala Leu Gly Ala Leu Ile Thr
        355                 360                 365

Arg Phe Pro Gly Leu Arg Leu Ala Gly Asp Ile Glu Trp Lys Asp Arg
    370                 375                 380

Met Leu Val Arg Gly Pro Arg Val Met Pro Ile Gly Trp
385                 390                 395

<210>  2
<211>  358
<212>  PRT
<213>  Streptomyces hygroscopicus

<400>  2

Met Pro Tyr Gly Glu Pro Ala Trp Leu Val Thr Arg Tyr Ala Asp Ala
1               5                   10                  15

Arg Leu Val Leu Gly Asp Arg Arg Phe Ser Arg Ala Glu Ala Leu His
            20                  25                  30

His Asp Glu Pro Arg Gln Ser Glu Gly Arg Arg Asp Ser Gly Ile Leu
        35                  40                  45

Thr Met Asp Pro Pro Asp His Thr Arg Leu Arg Thr Leu Val Ala Lys
    50                  55                  60

Ala Phe Thr Val His Gln Val Glu Lys Leu Arg Pro Trp Val Arg Gln
65                  70                  75                  80

Leu Thr His Asp Leu Leu Asp Asp Leu Glu Ala Ala Gly Pro Pro Ala
                85                  90                  95

Asp Leu Val Asp Arg Tyr Ala Leu Pro Ile Pro Val Gly Val Ile Cys
            100                 105                 110

Ala Met Leu Gly Val Pro Gln Glu Asp Arg Pro Lys Phe Arg Val Trp
        115                 120                 125

Ser Asp Ala Ala Leu Ser Thr Ser Ser Leu Ser Ala Glu Gln Phe Ala
    130                 135                 140

Arg Asn Thr Asp Glu Leu Arg Ala Tyr Met Ala Gly Leu Ile Glu Asp
145                 150                 155                 160

His Arg Arg Thr Pro Arg Asp Asp Ile Met Thr Ser Leu Ile Glu Ala
                165                 170                 175

Arg Asp Ala Gly Asp Arg Leu Ser Glu Leu Glu Leu Val Asp Leu Cys
            180                 185                 190

Val Gly Ile Leu Val Ala Gly His Glu Thr Thr Ala Thr Gln Ile Pro
        195                 200                 205

Asn Phe Val Leu Thr Leu Leu Glu His Pro Asp Gln Leu Arg Arg Leu
    210                 215                 220

Arg Glu Asp Pro Ala Leu Ile Gln Gly Ala Val Glu Glu Leu Leu Arg
225                 230                 235                 240

Phe Val Pro Leu Gly Val Gly Ala Ala Gln Ala Arg Tyr Ala Thr Glu
                245                 250                 255

Asp Ile Glu Val Gly Gly Thr Leu Val Arg Ser Gly Glu Pro Val Leu
            260                 265                 270

Val Ala Val Gly Ser Ala Asn Arg Asp Ala Leu Arg Phe Asp Glu Pro
        275                 280                 285

Gly Val Leu Asn Val Ala Arg Pro Thr Thr Gln His Leu Gly Phe Gly
    290                 295                 300

His Gly Val His His Cys Leu Gly Ala Pro Leu Ala Arg Leu Glu Leu
305                 310                 315                 320

Gln Glu Ala Leu Gly Ala Leu Ile Thr Arg Phe Pro Gly Leu Arg Leu
                325                 330                 335

Ala Gly Asp Ile Glu Trp Lys Asp Arg Met Leu Val Arg Gly Pro Arg
            340                 345                 350

Val Met Pro Ile Gly Trp
        355

<210>  3
<211>  1194
<212>  DNA
<213>  Streptomyces hygroscopicus

<400>  3
atgttgacca cagccgagac gacatccatc gcctatccct tcaacaccgc cgaagggctg        60

gcgctcagcg agcgttacga agaggccagg aaccgcaccg gactgctccg ggtgcggatg       120

ccctacggtg agcccgcctg gctggtcacg cggtacgccg acgcccggct ggtgctcggc       180

gaccggcgct tcagccgtgc ggaggcgctc caccacgacg agccgcggca gtccgaaggc       240

cggcgcgaca gcggcatcct gaccatggac ccgcccgacc acacccggct gcgcaccctc       300

gtcgccaagg cgttcaccgt ccaccaggtg gagaaactcc gcccctgggt acgccagttg       360

acccatgacc tgctcgacga cctcgaggcc gccgggccgc ccgccgatct ggtggaccgc       420

tacgccctgc ccattccggt cggcgtcatc tgcgccatgc tcggcgtccc gcaggaggac       480

cggcccaagt tccgggtctg gagcgacgcc gcgctgtcca ccagctcgct gagcgccgag       540

cagttcgccc gtaacaccga cgagctgcgc gcctacatgg ccgggctgat cgaggaccac       600

cgcaggaccc cgcgggacga catcatgacc tcgctgatcg aggcgcggga cgcgggcgac       660

cggctgtccg agctggaact cgtcgatctg tgcgtgggca tcctggtggc cgggcacgag       720

accaccgcca cccagatccc caacttcgtg ctgacgctgc tggagcaccc ggaccagctg       780

cgccggctgc gcgaggaccc cgccctgatc cagggcgccg tcgaggagct gctgcgcttc       840

gtcccgctgg gcgtgggcgc cgcccaggcc cgttacgcca ccgaggacat cgaggtgggc 900

ggcacgctgg tgcgcagcgg ggagccggtg ctggtcgccg tcggctcggc caaccgcgac 960

gcgctgcgct tcgacgaacc gggcgtgctc aacgtcgccc gccccaccac ccagcacctc 1020

ggcttcggcc acggtgtgca ccactgcctg ggcgcgcccc tggcccgtct ggagctccag 1080

gaggcgctcg gcgcgctgat cacgcgcttc ccgggcctgc ggctggccgg ggacatcgag 1140

tggaaggacc gcatgctggt ccgcgggccc cgtgtcatgc ccatcgggtg gtga 1194

<210> 4
<211> 1077
<212> DNA
<213> Streptomyces hygroscopicus

<400> 4
atgccctacg gtgagcccgc ctggctggtc acgcggtacg ccgacgcccg gctggtgctc 60

ggcgaccggc gcttcagccg tgcggaggcg ctccaccacg acgagccgcg gcagtccgaa 120

ggccggcgcg acagcggcat cctgaccatg gacccgcccg accacacccg gctgcgcacc 180

ctcgtcgcca aggcgttcac cgtccaccag gtggagaaac tccgcccctg ggtacgccag 240

ttgacccatg acctgctcga cgacctcgag gccgccgggc cgcccgccga tctggtggac 300

cgctacgccc tgcccattcc ggtcggcgtc atctgcgcca tgctcggcgt cccgcaggag 360

gaccggccca agttccgggt ctggagcgac gccgcgctgt ccaccagctc gctgagcgcc 420

gagcagttcg cccgtaacac cgacgagctg cgcgcctaca tggccgggct gatcgaggac 480

caccgcagga ccccgcggga cgacatcatg acctcgctga tcgaggcgcg ggacgcgggc 540

gaccggctgt ccgagctgga actcgtcgat ctgtgcgtgg gcatcctggt ggccgggcac 600

gagaccaccg ccacccagat ccccaacttc gtgctgacgc tgctggagca cccggaccag 660

ctgcgccggc tgcgcgagga ccccgccctg atccagggcg ccgtcgagga gctgctgcgc 720

ttcgtcccgc tgggcgtggg cgccgcccag gcccgttacg ccaccgagga catcgaggtg 780

ggcggcacgc tggtgcgcag cggggagccg gtgctggtcg ccgtcggctc ggccaaccgc 840

gacgcgctgc gcttcgacga accgggcgtg ctcaacgtcg cccgccccac cacccagcac 900

ctcggcttcg gccacggtgt gcaccactgc ctgggcgcgc ccctggcccg tctggagctc 960

caggaggcgc tcggcgcgct gatcacgcgc ttcccgggcc tgcggctggc cggggacatc 1020

gagtggaagg accgcatgct ggtccgcggg ccccgtgtca tgcccatcgg gtggtga 1077

<210> 5
<211> 1194
<212> DNA
<213> Künstliche Sequenz

<220>

<223>  E.coli Codon-optimiert SEQ ID Nr. 3

<400>  5
atgctgacca ccgcagaaac caccagtatt gcatatccgt ttaataccgc agaaggtctg      60

gcactgagcg aacgttatga agaagcacgt aatcgtaccg gtctgctgcg tgttcgtatg     120

ccgtatggtg aaccggcatg gctggttacc cgttatgcag atgcccgtct ggttctgggt     180

gatcgtcgtt ttagccgtgc cgaagcactg catcacgatg aaccgcgtca gagcgaaggt     240

cgtcgtgata gcggtattct gaccatggat ccgcctgatc atacccgtct gcgtaccctg     300

gttgcaaaag catttaccgt tcatcaggtt gaaaaactgc gtccgtgggt tcgccagctg     360

acccatgatc tgctggatga tctggaagca gcaggtccgc ctgcagatct ggttgatcgt     420

tatgcactgc cgattccggt tggtgttatt tgtgcaatgc tgggtgttcc gcaagaagat     480

cgtcctaaat ttcgtgtttg gagtgatgca gcactgagca ccagcagcct gagcgcagaa     540

cagtttgcac gtaataccga tgaactgcgt gcatatatgg caggtctgat tgaagatcat     600

cgtcgtacac cgcgtgatga tattatgacc agcctgatcg aagcacgtga tgccggtgat     660

cgcctgagtg aactggaact ggtggatctg tgtgttggta ttctggttgc aggtcatgaa     720

accaccgcaa cccagattcc gaattttgtt ctgaccctgc tggaacatcc ggatcagctg     780

cgtcgtctgc gtgaagatcc ggcactgatt cagggtgcag ttgaagaact gctgcgtttt     840

gttccgctgg gtgtgggtgc agcacaggca cgttatgcaa ccgaagatat tgaagttggt     900

ggcaccctgg ttcgtagtgg cgaaccggtg ctggttgccg ttggtagcgc aaaccgtgat     960

gcactgcgct ttgatgaacc gggtgttctg aatgttgcac gtccgaccac acagcatctg    1020

ggttttggtc atggtgttca tcattgtctg ggtgcaccgc tggcacgtct ggaactgcaa    1080

gaagcactgg gagcactgat tacccgtttt ccgggtctgc gtctggcagg cgatattgaa    1140

tggaaagatc gtatgctggt tcgtggtccg cgtgttatgc cgattggttg gtaa          1194


<210>  6
<211>  1070
<212>  DNA
<213>  Künstliche Sequenz

<220>
<223>  E.coli Codon-optimiert SEQ ID Nr. 4

<400>  6
atggtgaacc ggcatggctg gttacccgtt atgcagatgc ccgtctggtt ctgggtgatc      60

gtcgttttag ccgtgccgaa gcactgcatc acgatgaacc gcgtcagagc gaaggtcgtc     120

gtgatagcgg tattctgacc atggatccgc ctgatcatac ccgtctgcgt accctggttg     180

caaaagcatt taccgttcat caggttgaaa aactgcgtcc gtgggttcgc cagctgaccc     240

atgatctgct ggatgatctg gaagcagcag gtccgcctgc agatctggtt gatcgttatg     300

cactgccgat tccggttggt gttatttgtg caatgctggg tgttccgcaa gaagatcgtc 360

ctaaatttcg tgtttggagt gatgcagcac tgagcaccag cagcctgagc gcagaacagt 420

ttgcacgtaa taccgatgaa ctgcgtgcat atatggcagg tctgattgaa gatcatcgtc 480

gtacaccgcg tgatgatatt atgaccagcc tgatcgaagc acgtgatgcc ggtgatcgcc 540

tgagtgaact ggaactggtg gatctgtgtg ttggtattct ggttgcaggt catgaaacca 600

ccgcaaccca gattccgaat tttgttctga ccctgctgga acatccggat cagctgcgtc 660

gtctgcgtga agatccggca ctgattcagg gtgcagttga agaactgctg cgttttgttc 720

cgctgggtgt gggtgcagca caggcacgtt atgcaaccga agatattgaa gttggtggca 780

ccctggttcg tagtggcgaa ccggtgctgg ttgccgttgg tagcgcaaac cgtgatgcac 840

tgcgctttga tgaaccgggt gttctgaatg ttgcacgtcc gaccacacag catctgggtt 900

ttggtcatgg tgttcatcat tgtctgggtg caccgctggc acgtctggaa ctgcaagaag 960

cactgggagc actgattacc cgttttccgg gtctgcgtct ggcaggcgat attgaatgga 1020

aagatcgtat gctggttcgt ggtccgcgtg ttatgccgat tggttggtaa 1070

<210> 7
<211> 107
<212> PRT
<213> Pseudomonas putida

<220>
<221> MISC_FEATURE
<222> (1)..(1)
<223> Xaa ist Methionin oder keine Aminosäure

<400> 7

Xaa Ser Lys Val Val Tyr Val Ser His Asp Gly Thr Arg Arg Glu Leu
1               5                   10                  15

Asp Val Ala Asp Gly Val Ser Leu Met Gln Ala Ala Val Ser Asn Gly
            20                  25                  30

Ile Tyr Asp Ile Val Gly Asp Cys Gly Gly Ser Ala Ser Cys Ala Thr
        35                  40                  45

Cys His Val Tyr Val Asn Glu Ala Phe Thr Asp Lys Val Pro Ala Ala
    50                  55                  60

Asn Glu Arg Glu Ile Gly Met Leu Glu Cys Val Thr Ala Glu Leu Lys
65                  70                  75                  80

Pro Asn Ser Arg Leu Cys Cys Gln Ile Ile Met Thr Pro Glu Leu Asp
                85                  90                  95

Gly Ile Val Val Asp Val Pro Asp Arg Gln Trp
            100                 105

<210> 8
<211> 422
<212> PRT
<213> Pseudomonas putida

<400> 8

Met Asn Ala Asn Asp Asn Val Val Ile Val Gly Thr Gly Leu Ala Gly
1               5                   10                  15

Val Glu Val Ala Phe Gly Leu Arg Ala Ser Gly Trp Glu Gly Asn Ile
            20                  25                  30

Arg Leu Val Gly Asp Ala Thr Val Ile Pro His His Leu Pro Pro Leu
        35                  40                  45

Ser Lys Ala Tyr Leu Ala Gly Lys Ala Thr Ala Glu Ser Leu Tyr Leu
    50                  55                  60

Arg Thr Pro Asp Ala Tyr Ala Ala Gln Asn Ile Gln Leu Leu Gly Gly
65                  70                  75                  80

Thr Gln Val Thr Ala Ile Asn Arg Asp Arg Gln Gln Val Ile Leu Ser
                85                  90                  95

Asp Gly Arg Ala Leu Asp Tyr Asp Arg Leu Val Leu Ala Thr Gly Gly
            100                 105                 110

Arg Pro Arg Pro Leu Pro Val Ala Ser Gly Ala Val Gly Lys Ala Asn
        115                 120                 125

Asn Phe Arg Tyr Leu Arg Thr Leu Glu Asp Ala Glu Cys Ile Arg Arg
    130                 135                 140

Gln Leu Ile Ala Asp Asn Arg Leu Val Val Ile Gly Gly Gly Tyr Ile
145                 150                 155                 160

Gly Leu Glu Val Ala Ala Thr Ala Ile Lys Ala Asn Met His Val Thr
                165                 170                 175

Leu Leu Asp Thr Ala Ala Arg Val Leu Glu Arg Val Thr Ala Pro Pro
            180                 185                 190

Val Ser Ala Phe Tyr Glu His Leu His Arg Glu Ala Gly Val Asp Ile
        195                 200                 205

Arg Thr Gly Thr Gln Val Cys Gly Phe Glu Met Ser Thr Asp Gln Gln
    210                 215                 220

Lys Val Thr Ala Val Leu Cys Glu Asp Gly Thr Arg Leu Pro Ala Asp
225                 230                 235                 240

Leu Val Ile Ala Gly Ile Gly Leu Ile Pro Asn Cys Glu Leu Ala Ser
                245                 250                 255

Ala Ala Gly Leu Gln Val Asp Asn Gly Ile Val Ile Asn Glu His Met
            260                 265                 270

Gln Thr Ser Asp Pro Leu Ile Met Ala Val Gly Asp Cys Ala Arg Phe
        275                 280                 285

His Ser Gln Leu Tyr Asp Arg Trp Val Arg Ile Glu Ser Val Pro Asn
    290                 295                 300

Ala Leu Glu Gln Ala Arg Lys Ile Ala Ala Ile Leu Cys Gly Lys Val
305                 310                 315                 320

Pro Arg Asp Glu Ala Ala Pro Trp Phe Trp Ser Asp Gln Tyr Glu Ile
                325                 330                 335

Gly Leu Lys Met Val Gly Leu Ser Glu Gly Tyr Asp Arg Ile Ile Val
            340                 345                 350

Arg Gly Ser Leu Ala Gln Pro Asp Phe Ser Val Phe Tyr Leu Gln Gly
        355                 360                 365

Asp Arg Val Leu Ala Val Asp Thr Val Asn Arg Pro Val Glu Phe Asn
    370                 375                 380

Gln Ser Lys Gln Ile Ile Thr Asp Arg Leu Pro Val Glu Pro Asn Leu
385                 390                 395                 400

Leu Gly Asp Glu Ser Val Pro Leu Lys Glu Ile Ile Ala Ala Ala Lys
                405                 410                 415

Ala Glu Leu Ser Ser Ala
            420

<210>  9
<211>  324
<212>  DNA
<213>  Pseudomonas putida

<220>
<221>  misc_feature
<222>  (1)..(1)
<223>  n ist A oder kein Nukleotid

<220>
<221>  misc_feature
<222>  (2)..(2)
<223>  n ist T oder kein Nukleotid

<220>
<221>  misc_feature
<222>  (3)..(3)
<223>  n ist G oder kein Nukleotid

<400>  9
nnntctaaag tagtgtatgt gtcacatgat ggaacgcgtc gcgaactgga tgtggcggat      60

ggcgtcagcc tgatgcaggc tgcagtctcc aatggtatct acgatattgt cggtgattgt     120

ggcggcagcg ccagctgtgc cacctgccat gtctatgtga acgaagcgtt cacggacaag     180

gtgcccgccg ccaacgagcg ggaaatcggc atgctggagt gcgtcacggc cgaactgaag     240

ccgaacagca ggctctgctg ccagatcatc atgacgcccg agctggatgg catcgtggtc     300

gatgttcccg ataggcaatg gtaa                                            324


<210>  10
<211>  1269
<212>  DNA
<213>  Pseudomonas putida

<400>  10
atgaacgcaa acgacaacgt ggtcatcgtc ggtaccggac tggctggcgt tgaggtcgcc      60

ttcggcctgc gcgccagcgg ctgggaaggc aatatccggt tggtggggga tgcgacggta     120

attccccatc acctaccacc gctatccaaa gcttacttgg ccggcaaagc cacagcggaa     180

agcctgtacc tgagaacccc agatgcctat gcagcgcaga acatccaact actcggaggc     240

acacaggtaa cggctatcaa ccgcgaccga cagcaagtaa tcctatcgga tggccgggca     300

ctggattacg accggctggt attggctacc ggagggcgtc caagacccct accggtggcc     360

agtggcgcag ttggaaaggc gaacaacttt cgatacctgc gcacactcga ggacgccgag     420

tgcattcgcc ggcagctgat tgcggataac cgtctggtgg tgattggtgg cggctacatt     480

ggccttgaag tggctgccac cgccatcaag gcgaacatgc acgtcaccct gcttgatacg     540

gcagcccggg ttctggagcg ggttaccgcc ccgccggtat cggcctttta cgagcaccta     600

caccgcgaag ccggcgttga catacgaacc ggcacgcagg tgtgcgggtt cgagatgtcg     660

accgaccaac agaaggttac tgccgtcctc tgcgaggacg gcacaaggct gccagcggat     720

ctggtaatcg ccgggattgg cctgatacca aactgcgagt tggccagtgc ggccggcctg     780

caggttgata acggcatcgt gatcaacgaa cacatgcaga cctctgatcc cttgatcatg     840

gccgtcggcg actgtgcccg atttcacagt cagctctatg accgctgggt gcgtatcgaa 900

tcggtgccca atgccttgga gcaggcacga aagatcgccg ccatcctctg tggcaaggtg 960

ccacgcgatg aggcggcgcc ctggttctgg tccgatcagt atgagatcgg attgaagatg 1020

gtcggactgt ccgaagggta cgaccggatc attgtccgcg gctctttggc gcaacccgac 1080

ttcagcgttt tctacctgca gggagaccgg gtattggcgg tcgatacagt gaaccgtcca 1140

gtggagttca accagtcaaa acaaataatc acggatcgtt tgccggttga accaaaccta 1200

ctcggtgacg aaagcgtgcc gttaaaggaa atcatcgccg ccgccaaagc tgaactgagt 1260

agtgcctga 1269

<210> 11
<211> 327
<212> DNA
<213> Künstliche Sequenz

<220>
<223> E.coli Codon-optimiert SEQ ID Nr. 9

<220>
<221> misc_feature
<222> (1)..(1)
<223> n ist A oder kein Nukleotid

<220>
<221> misc_feature
<222> (2)..(2)
<223> n ist T oder kein Nukleotid

<220>
<221> misc_feature
<222> (3)..(3)
<223> n ist G oder kein Nukleotid

<400> 11
nnnatgagca aagtggtcta tgtgtcgcat gatggaacac gccgtgagtt agacgtcgct 60

gatggtgtat ccctgatgca agcagcggtt agcaatggca tttacgacat cgttggcgat 120

tgtggtggta gtgcgtcatg tgcaacgtgt cacgtgtatg ttaacgaagc gtttaccgat 180

aaggtgcctg ctgccaatga acgcgagatt ggcatgctgg aatgcgtaac tgccgaactc 240

aaaccgaact ctcgcctgtg ctgccagatc atcatgaccc cggaattgga cgggattgtc 300

gttgatgtgc cagatcgtca gtggtaa 327

<210> 12
<211> 1269
<212> DNA
<213> Künstliche Sequenz

<220>
<223> E.coli Codon-optimiert SEQ ID Nr. 10

<400> 12
atgaacgcca atgataatgt tgttattgtt ggcaccggtc tggcaggcgt tgaagttgca 60

tttggtctgc gtgcaagcgg ttgggaaggt aatattcgtc tggttggtga tgcaaccgtt 120

attccgcatc atctgcctcc gctgagcaaa gcatatctgg caggtaaagc aaccgcagaa 180

agcctgtatc tgcgtacacc ggatgcctat gcagcacaga atattcagct gctgggtggt 240

acacaggtta ccgcaattaa tcgtgatcgt cagcaggtta ttctgagtga tggtcgtgca 300

ctggattatg atcgtctggt gctggcaacc ggtggtcgtc cgcgtccgct gccggttgca 360

agtggtgcag ttggtaaagc caataacttt cgttatctgc gcaccctgga agatgcagaa 420

tgtattcgtc gtcagctgat tgcagataat cgcctggttg tgattggtgg tggttatatt 480

ggtctggaag ttgcagcaac cgccattaaa gcaaatatgc atgttaccct gctggatacc 540

gcagcacgtg ttctggaacg tgttaccgca ccgcctgtta gcgcctttta tgaacatctg 600

catcgtgaag ccggtgttga tattcgtacc ggcacccagg tttgtggttt tgaaatgagc 660

accgatcagc agaaagttac cgcagttctg tgtgaagatg gcacccgtct gcctgcagat 720

ctggttattg caggtattgg cctgattccg aattgtgaac tggcaagcgc agcaggtctg 780

caggttgata atggtattgt tattaacgaa cacatgcaga ccagcgatcc gctgattatg 840

gcagttggtg attgtgcacg ttttcatagc cagctgtatg atcgttgggt tcgtattgaa 900

agcgttccga atgcactgga acaggcacgt aaaattgcag caattctgtg tggtaaagtt 960

ccgcgtgatg aagcagcacc gtggttttgg agcgatcagt atgaaattgg tctgaaaatg 1020

gttggtctga gcgaaggtta tgatcgcatt attgttcgtg gtagcctggc acagccggat 1080

ttttcagttt tttatctgca gggtgatcgt gtgctggcag ttgataccgt taatcgtccg 1140

gttgaattta accagagcaa acaaattatc accgatcgtc tgccggtgga accgaatctg 1200

ctgggagatg aaagcgtgcc gctgaaagaa attattgcag cagcaaaagc agaactgagc 1260

agcgcataa 1269